# EUROPEAN PATENT APPLICATION

(11) **EP 4 257 686 A1**
(43) Date of publication of application: **11.10.2023**
(21) Application number: 21903785.0
(22) Date of filing: 06.12.2021
(51) Int. Cl.: C12N 15/113, A61K 31/7088, A61P 27/02

(54) **NUCLEIC ACID MOLECULE FOR INDUCTION OF ASYMMETRIC RNAI FOR INHIBITING EXPRESSION OF ROR-BETA**

(30) Priority: 07.12.2020 KR 20200169855
(71) Applicant: OliX Pharmaceuticals, Inc., Gyeonggi-do 16226 (KR)
(72) Inventor: CHUNG, Hye Won, Seoul 06320 (KR); PARK, June Hyun, Suwon-si, Gyeonggi-do 16281 (KR); YANG, Seung Ya, Seongnam-si, Gyeonggi-do 13607 (KR)
(74) Representative: Weickmann & Weickmann PartmbB
(86) International application number: PCT/KR2021/018386
(87) International publication number: WO 2022/124739

(57) **Abstract**

The present application relates to an asymmetric RNAi-inducing nucleic acid molecule which inhibits the expression of retinoid-related orphan nuclear receptor-beta (ROR-beta) and use thereof, and more particularly to an asymmetric RNAi-inducing nucleic acid molecule comprising an antisense strand comprising a sequence complementary to mRNA encoding ROR-beta, and a sense strand forming complementary bonds with the antisense strand, and a pharmaceutical composition for ameliorating or treating a retinal disease, comprising the asymmetric RNAi-inducing nucleic acid molecule.

## Description

### Technical Field

The present disclosure relates to an asymmetric RNAi(RNA interference)-inducing nucleic acid molecule, which inhibits the expression of retinoid-related orphan nuclear receptor (ROR)-beta and use thereof, and more particularly to an asymmetric RNAi-inducing nucleic acid molecule comprising an antisense strand comprising a sequence complementary to mRNA encoding ROR-beta, and a sense strand forming complementary bonds with the antisense strand; and a pharmaceutical composition for ameliorating or treating retinal diseases, comprising the asymmetric RNAi-inducing nucleic acid molecule.

### Background Art

Retinitis pigmentosa is a disease in which genes that give characteristics of rod cells are mutated, leading to destruction of rod cells and secondary destruction of cone cells, which may eventually lead to blindness. Retinoid-related orphan nuclear receptor (ROR)-beta is a transcription factor that plays an important role in the differentiation of rod cells and cone cells, regulates the expression of neural retina leucine zipper (NRL) that regulates the differentiation into rod cells, and is involved in various retinal functions. ROR-beta has two isoforms of RORβ1 and RORβ2 in which RORβ1 induces NRL expression upstream of NRL, and RORβ2, which is a factor regulated by NRL, enhances NRL expression. That is, a positive feedback action occurs between the two genes of ROR-beta and NRL, stabilizing rod cell differentiation. (Fu et al., "Feedback Induction of a Photoreceptor-specific Isoform of Retinoid-related Orphan Nuclear Receptor β by the Rod Transcription Factor NRL" Journal of Biological Chemistry 2014 Nov 21;289(47):32469-80.) In particular, it was observed through a retinal degeneration mouse model that, in the case of NRL, which is closely related to ROR-beta, when knocked out with adenovirus-associated virus (AAV) delivered CRISPR/Cas9, rod cells partially acquire the characteristics of cone cells, and thus, when genes that give the characteristics of rod cells are mutated, the rod cells are not destroyed and are maintained, thus preventing the secondary loss of cone cells (Yu, Wenhan, et al., "Nr1 knockdown by AAV-delivered CRISPR/Cas9 prevents retinal degeneration in mice." Nature communications 8 (2017): 14716). This phenomenon is similar to studies in which rod cells acquire the characteristics of cone cells in ROR-beta-deficient mice (jia et al., "Retinoid-related orphan nuclear receptor RORβ is an early-acting factor in rod photoreceptor development" Proceedings of the National Academy of Sciences of the United States of America (2009) 13;106(41):17534-9.), and thus it can be seen that NRL and ROR-beta play very important roles in the differentiation process of rod cells and cone cells in the retina.

Meanwhile, since the treatment of diseases by RNA interference uses siRNA that targets mRNA and regulates gene expression at a translational level, diseases can be treated more safely. Small interfering RNA (siRNA) consists of a sense strand having the same sequence as target mRNA and an antisense strand having a sequence complementary thereto. Conventional siRNA has a short duplex of 19-21 bp, with two nucleotides protruding from the 3' ends of both strands. siRNA enters a cell, attaches to a target mRNA, degrades the target mRNA, and suppresses the expression of a target gene. Since it is possible to target all mRNAs by changing the sequence of oligos, the expression of proteins with complex structures can also be inhibited, and thus diseases such as cancer, viral infection, and genetic diseases, which are currently intractable, can be treated. However, siRNA introduced into cells may cause side effects such as induction of immune responses and suppression of off-target genes. Among these, a delivery system that introduces siRNA into cells has become the biggest problem in the development of therapeutic agents using siRNA.

siRNA is negatively charged due to the phosphate backbone and has a repulsive force against the negatively charged cell membrane, and thus a delivery system is required to introduce siRNA into a cell. As an example of the delivery system, a method of encapsulating siRNA with a positively charged liposome or polymer to offset the negative charge and introducing the siRNA into a cell is widely used. However, positively charged carriers may cause various side effects, such as exhibiting unwanted toxicity through attachment to a negatively charged cell membrane, or formation of unwanted complexes through interactions with various types of proteins. In addition, since siRNA is rapidly degraded by nucleases in the blood, the amount of siRNA reaching a target cell may not be sufficient to significantly reduce the expression of a target gene. Therefore, there is a need for a method that can safely and effectively deliver siRNA into target cells.

Therefore, as a result of having intensively studied and made efforts to select ROR-beta-targeting siRNAs capable of inhibiting ROR-beta expression, and develop siRNAs that are delivered into a cell without the aid of a carrier and are highly resistant to nucleases, the inventors of the present disclosure designed siRNAs targeting ROR-beta, and selected siRNAs that inhibit ROR-beta most efficiently through screening, and confirmed that intracellular delivery problems can be overcome through modifications, thus completing the present disclosure.

The information described in the Background Art section is only for improving understanding of the background of the present disclosure, and thus may not include information that forms prior art known to those of ordinary skill in the art to which the present disclosure pertains.

### Disclosure

### Technical Problem

An object of the present disclosure is to provide an asymmetric RNAi-inducing nucleic acid molecule, which specifically inhibits the expression of ROR-beta.

Another object of the present disclosure is to provide a pharmaceutical composition for ameliorating or treating a retinal disease, comprising the asymmetric RNAi-inducing nucleic acid molecule, or a method of ameliorating or treating a retinal disease.

### Technical Solution

To achieve the above object, the present disclosure provides an RNAi-inducing nucleic acid molecule comprising an antisense strand which includes a sequence complementary to mRNA encoding retinoid-related orphan nuclear receptor (ROR)-beta, and a sense strand which forms complementary bonds with the antisense strand, wherein the 5' end of the antisense strand and the 3' end of the sense strand form a blunt end.

The present disclosure also provides a pharmaceutical composition for ameliorating or treating a retinal disease, including the RNAi-inducing nucleic acid molecule.

The present disclosure provides a method for ameliorating or treating a retinal disease, including administering the RNAi-inducing nucleic acid molecule to a subject.

### Advantageous Effects

According to the present disclosure, asymmetric siRNA capable of efficiently inhibiting the expression of ROR-beta, which plays a very important role in the differentiation process of rod cells and cone cells, is selected, and the siRNA is chemically modified to be introduced into a cell without the aid of a carrier and to be resistant to nucleases, thereby removing cytotoxicity due to a carrier and enabling more efficient inhibition of gene expression *in vivo,* and thus the siRNA can be effectively used as a therapeutic agent for retinal diseases, including retinitis pigmentosa.

### Description of Drawings

FIG. 1 is a schematic view illustrating a structure of ROR-beta asiRNA consisting of a 16-mer sense strand and a 19-mer antisense strand.
FIG. 2 illustrates the results of confirming changes in ROR-beta protein expression, after Y-79 cells were treated with 62 types of ROR-beta asiRNA.
FIG. 3 illustrates the results of confirming changes in ROR-beta protein expression, after Y-79 cells were treated with 13 types of ROR-beta asiRNA.
FIG. 4 illustrates the results of confirming changes in ROR-beta mRNA expression, after Y-79 cells were treated with 13 types of ROR-beta asiRNA.
FIG. 5 illustrates the results of confirming changes in ROR-beta protein expression, after Y-79 cells were treated with 67 types of ROR-beta cp-asiRNA.
FIG. 6 illustrates the results of confirming changes in ROR-beta protein expression, after Y-79 cells were treated with 10 types of ROR-beta cp-asiRNA.
FIG. 7 illustrates the results of confirming changes in ROR-beta protein expression, after A549 cells transiently expressing ROR-beta by using a ROR-beta plasmid were treated with 10 types of cp-asiRNA.
FIG. 8 illustrates the results of confirming changes in ROR-beta mRNA expression, after A549 cells transiently expressing ROR-beta by using a ROR-beta plasmid were treated with 5 types of cp-asiRNA.
FIG. 9 illustrates the results of confirming changes in ROR-beta protein expression, after A549 cells transiently expressing ROR-beta by using a ROR-beta plasmid were treated with 5 types of cp-asiRNA.
FIG. 10 illustrates the sequence and modification information of ROR-beta cp-asiRNA 4 (OLX304C-026-4) and ROR-beta cp-asiRNA 5 (OLX304C-026-5) (sense strand: 16 mer, antisense strand: 19 mer).
FIG. 11 illustrates the results of confirming changes in ROR-beta protein expression, after normal mouse eyes were treated with 2 types of ROR-beta cp-asiRNA (OLX304C-026-4 and OLX304C-026-5), in which (A) illustrates the results of confirming changes in ROR-beta protein expression after retinal tissues from normal mice were isolated, and (B) illustrates the results of confirming changes in ROR-beta protein expression after retinal pigment epithelial cells/choroid layer of normal mice were isolated.
FIG. 12 illustrates the results of confirming changes in ROR-beta mRNA expression according to dose in retinal tissue, after normal mouse eyes were treated with two types of ROR-beta cp-asiRNA (OLX304C-026-4 and OLX304C-026-26) according to dose.
FIG. 13 illustrates the results of confirming the duration of the expression inhibitory effect of ROR-beta mRNA according to dose in retinal tissue, after normal mouse eyes were treated with one type of ROR-beta cp-asiRNA (OLX304C-026-4) according to dose.
FIG. 14 illustrates the results of comparing the thicknesses of respective layers through H&E staining of eye tissue, after a retinitis pigmentosa-related human transgenic mouse model was treated with two types of ROR-beta cp-asiRNA (OLX304C-026-4 and OLX304C-026-26) at the optimal dose.
FIG. 15 illustrates electroretinogram (ERG) results obtained after a retinitis pigmentosa-related human transgenic mouse model was treated with two types of ROR-beta cp-asiRNA (OLX304C-026-4 and OLX304C-026-26) at the optimal dose, in which (A) illustrates the results of confirming changes in a-wave amplitude, and (B) illustrates the results of confirming changes in b-wave amplitude.

### Best Mode

### Mode for Invention

Unless defined otherwise, all technical and scientific terms used herein have the same meaning as commonly understood by one of ordinary skill in the art to which the present disclosure pertains. Generally, the nomenclature used herein is well known in the art and commonly used.

Definitions of main terms used in the detailed description of the present disclosure are as follows.

The term "RNA interference (RNAi)" refers to a mechanism that inhibits the expression of a target gene by inducing the degradation of mRNA of the target gene by introducing double-stranded RNA (dsRNA) consisting of a strand having a sequence homologous to mRNA of the target gene and a strand having a sequence complementary thereto.

The term "RNAi-inducing nucleic acid molecules" refers to any nucleic acid molecule capable of inhibiting or down-regulating gene expression or viral replication by mediating the RNA interference in a sequence-specific manner. The terms may refer to an individual nucleic acid molecule, a plurality of the nucleic acid molecules, or a pool of the nucleic acid molecules. In an embodiment, the RNAi-inducing nucleic acid molecule may be siRNA.

The term "small interfering RNA (siRNA)" refers to a short double-stranded RNA (dsRNA) that mediates sequence-specific efficient gene silencing.

The term "antisense strand" is a polynucleotide that is substantially or 100% complementary to a target nucleic acid of interest, and may be complementary, in whole or in part, to, for example, messenger RNA (mRNA), non-mRNA RNA sequences (e.g., microRNA, piwiRNA, tRNA, rRNA, and hnRNA), or encoding or non-encoding DNA sequences.

The term "sense strand" is a polynucleotide that has a nucleic acid sequence identical to a target nucleic acid, and refers to a polynucleotide that is identical to, in whole or in part, messenger RNA (mRNA), non-mRNA RNA sequences (e.g., microRNA, piwiRNA, tRNA, rRNA, and hnRNA), or encoding or non-encoding DNA sequences.

The term "gene" should be considered in the broadest sense, and may encode a structural protein or a regulatory protein. In this regard, the regulatory protein includes a transcription factor, a heat shock protein, or a protein involved in DNA/RNA replication, transcription, and/or translation. In the present disclosure, the target gene of which expression is to be inhibited is resident in a viral genome, and may be integrated into an animal gene or may be present as an extrachromosomal element. For example, the target gene may be a gene on an HIV genome. In this case, siRNA molecules are useful in inactivating translation of the HIV gene in a mammalian cell.

The term "retinoid-related orphan nuclear receptor (ROR)-beta" is an important transcription factor that regulates the differentiation of rod cells and cone cells in the retina, and is known to act as an upstream regulator of neural retina leucine zipper (NRL) and nuclear receptor subfamily 2, group E, member 3 (NR2E3), which is a downstream factor thereof, which allows photoreceptor precursors to differentiate into rod cells. Meanwhile, it has been reported that, in ROR-beta gene-deficient *Rorb-*/*-* mice, Nr1 expression and Nr2e3 expression are remarkably reduced, and the expression of rod cell-related genes is reduced, whereas the expression of cone cell-related genes is increased, and therefore, ROR-beta and NRL play an important role in regulation of signaling of differentiation of rod cells.

Retinitis pigmentosa is a disease in which genes that give characteristics of rod cells are mutated, leading to primary destruction of rod cells and secondary destruction of cone cells. According to the research findings, the adverse effects of mutations in genes important for photoreceptor function are maximized in a fully differentiated photoreceptor environment. Thus, when ROR-beta, which is un upstream factor of NRL that induces differentiation into rod cells, is knocked out, rod cells are converted into cells morphologically similar to cone cells and lose their function. However, even rod cells with altered morphology and function have potential as a treatment for retinitis pigmentosa since the existence thereof prevents secondary loss of cone cells.

The object of the present disclosure may be broadly expressed in two aspect. The first object is to design siRNAs targeting ROR-beta, and select siRNAs that most efficiently inhibit ROR-beta through screening, and the second object is to introduce a chemical modification to deliver siRNA into cells without any specific carrier and increase resistance to nucleases. siRNA must be small or hydrophobic to pass through cell membranes consisting of phospholipids. However, siRNA is negatively charged by the phosphate backbone, and thus it is difficult for the siRNA to penetrate the cell membrane. In addition, it is necessary to increase resistance to nucleases for siRNA to have a long lifetime in serum such that the amount reaching a target may be sufficient to induce effective RNAi. Therefore, by introducing modifications, the siRNA delivery problem was overcome.

In an embodiment of the present invention, first, ROR-beta-targeting asiRNA was designed, and ROR-beta-expressing cells were transfected with the asiRNA to select ROR-beta asiRNA with the best knockdown efficiency. The following four modifications are introduced into the selected siRNA so that asiRNA has cell-penetrating ability and resistance to nucleases. First, cholesterol is added to the 3' end of the sense strand to allow siRNA to penetrate the cell membrane. Second, by substituting the phosphate backbone near the 5' end or 3' end of the sense strand and antisense strand with phosphorothioate, siRNA has resistance to exonucleases, and uptake into cells and bioavailability of siRNA *in vivo* are possible. Third, by modifying the 2' of sugar with OMethyl, resistance to nucleases is obtained, siRNA immunogenicity is lowered, and off-target effects are reduced. Fourth, the 2' of sugar is modified with fluoro to impart stability to a double strand duplex, thus increasing stability in serum and enabling efficient silencing *in vitro* and *in vivo.* By applying the above modification to siRNA, siRNA has cell-penetrating ability and stays in serum for a longer period of time, and thus a sufficient amount of siRNA is delivered to target cells, thus enabling more sufficient gene suppression.

Therefore, an aspect of the present disclosure relates to an RNAi-inducing nucleic acid molecule including: an antisense strand including a sequence complementary to mRNA encoding retinoid-related orphan nuclear receptor (ROR)-beta; and a sense strand that forms a complementary bond with the antisense strand, in which the 5' end of the antisense strand and the 3' end of the sense strand form a blunt end.

siRNA in the present disclosure is a concept that includes all substances having a general RNA interference (RNAi) action. RNAi is an intracellular gene regulation mechanism first discovered in Caenorthabditis elegans in 1998, and the mechanism of action is known in that an antisense strand of RNA double strands introduced into a cell complementarily binds to the mRNA of a target gene to thereby induce target gene degradation. Among these, siRNA is one of the methods to suppress gene expression "in *vitro."* siRNA having 19-21 bp can theoretically selectively inhibit almost all genes, and thus can be developed as a therapeutic agent for various gene-related diseases such as cancer and viral infection, and is the most popular new drug development candidate technology. In mid-2003, *in vivo* treatment with siRNA was first attempted in mammals, and since then, there have been numerous reports on *in vivo* treatment as a result of many attempts at applied research.

However, contrary to the possibility, side effects and disadvantages of siRNA have been continuously reported. To develop RNAi-based therapeutic agents, it is necessary to overcome barriers such as: 1) lack of an effective delivery system 2) off-target effect 3) immune response induction 4) saturation of intracellular RNAi mechanisms. Due to these problems, although siRNA is an effective method to directly control the expression of a target gene, there are difficulties in developing therapeutic agents. In this regard, asymmetric shorter duplex siRNA (asiRNA) is an asymmetric RNAi-inducing structure having a shorter duplex length than the 19+2 structure of conventional siRNAs. asiRNA is a technology that overcomes problems, such as off-target effects, saturation of the RNAi mechanism, and immune responses by TLR3, confirmed in existing siRNA structure technologies, and accordingly, it is possible to develop RNAi new drugs with fewer side effects.

On the basis of this, an embodiment provides an asymmetric siRNA including a sense strand and an antisense strand complementary to the sense strand, and the siRNA according to an embodiment may not cause problems such as off-target effects and saturation of RNAi mechanisms, and thus may effectively suppress ROR-beta target gene expression at a desired level while stably maintaining high delivery efficiency.

In the present disclosure, the sense strand of the RNAi-inducing nucleic acid molecule may have a length of 15 nt to 17 nt, and the antisense strand thereof may have a length of 18 nt or more. Although not limited thereto, the antisense strand may have a length of 18 nt to 31 nt, and preferably, may have a length of 18 nt to 23 nt. More preferably, the sense strand may have a length of 16 nt, and the antisense strand complementary thereto may have a length of 19 nt, 20 nt, 21 nt, or 22 nt, but the present disclosure is not limited thereto.

The 3' end of the sense strand and the 5' end of the antisense strand form a blunt end. A 3' end of the antisense strand may include, for example, an overhang of 1 nt to 16 nt.

In an embodiment of the present disclosure, 62 ROR-beta asiRNAs were designed to inhibit ROR-beta expression, and the suppression thereof at an mRNA level and a protein level in ROR-beta-expressing cells or cells transiently expressing ROR-beta was confirmed.

In the present disclosure, the sense strand may be selected from the group consisting of SEQ ID NOs:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, and 123.

Specifically, the sense strand may be, for example, selected from the group consisting of SEQ ID NOs: 27, 29, 51, 83, 85, 91, 95, 97, 103, 105, 107, 109, and 115 or selected from the group consisting of SEQ ID NOs: 27, 29, 51, and 109, and the sense strand may be, for example, SEQ ID NO: 51 or 109.

In the present disclosure, the antisense strand may be selected from the group consisting of SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, and 124.

Specifically, the antisense strand may be, for example, selected from the group consisting of SEQ ID NOs: 28, 30, 52, 84, 86, 92, 96, 98, 104, 106, 108, 110, and 116 or selected from the group consisting of SEQ ID NOs: 28, 30, 52, and 110, and the antisense strand may be, for example, SEQ ID NO: 52 or 110.

In the present disclosure, the sense strand or the antisense strand of the RNAi-inducing nucleic acid molecule may include at least one chemical modification.

General siRNA may not pass through the cell membrane due to high molecular weight and high negative charge due to the phosphate backbone structure, and it is difficult to deliver a sufficient amount for RNAi induction to an actual target site since general siRNA is rapidly degraded and removed from the blood. Currently, in the case of *in vitro* delivery, many high-efficiency delivery methods using cationic lipids and cationic polymers have been developed, but in the case of *in vivo* delivery, it is difficult to deliver siRNA with as high efficiency as *in vitro,* and there is a problem in that siRNA delivery efficiency decreases due to interactions with various proteins present *in vivo.*

Therefore, the inventors of the present disclosure have developed an asiRNA structure (cp-asiRNA) having an effective and self-delivering ability capable of intracellular delivery without a separate carrier by introducing chemical modifications into the asymmetric siRNA structure.

In the present disclosure, a chemical modification in the sense strand or the antisense strand may include at least one selected from the group consisting of: substitution of -OH group at a 2' carbon position of a sugar structure of a nucleotide with methyl (-CH₃), methoxy (-OCH₃), -NH₂, fluorine (-F), -O-2-methoxyethyl-O-propyl, -0-2-methylthioethyl, -O-3-aminopropyl, or -O-3-dimethylaminopropyl; substitution of oxygen of a sugar structure of a nucleotide with sulfur; modification of a nucleotide linkage with phosphorothioate, boranophosphate, or methyl phosphonate; modification into a peptide nucleic acid (PNA) form, a locked nucleic acid (LNA) form, or an unlocked nucleic acid (UNA) form; and linkage of a phosphate group, a lipophilic compound, or a cell-penetrating peptide.

In the present disclosure, the lipophilic compound may be selected from the group consisting of cholesterol, tocopherol, stearic acid, retinoic acid, decosahexaenoic acid (DHA), palmitic acid, linoleic acid, linolenic acid, and long-chain fatty acids having 10 or more carbon atoms. Preferably, the lipophilic compound may be cholesterol, but the present disclosure is not limited thereto.

In an embodiment, the sense strand may include at least one chemical modification selected from: modification of 2 to 4 nucleotide linkages adjacent to the 3' end with phosphorothioate, boranophosphate, or methyl phosphonate; substitution of an -OH group at a 2' carbon position of a sugar structure of two or more nucleotides with methyl (-CH₃), methoxy (-OCH₃), -NH₂, fluorine (-F), -O-2-methoxyethyl-O-propyl, -0-2-methylthioethyl, -O-3-aminopropyl, or -O-3-dimethylaminopropyl; and linkage of a lipophilic compound or a cell-penetrating peptide to the 3' end.

In an embodiment, the antisense strand may include at least one chemical modification selected from: modification of 3 to 5 nucleotide linkages adjacent to the 3' end with phosphorothioate, boranophosphate, or methyl phosphonate; substitution of - OH group at the 2' carbon position of a sugar structure of two or more nucleotides with methyl (-CH₃), methoxy (-OCH₃), -NH₂, fluorine (-F), -O-2-methoxyethyl-O-propyl, -0-2-methylthioethyl, -O-3-aminopropyl, or -O-3-dimethylaminopropyl; and linkage of a phosphate group or a cell-penetrating peptide to the 5' end.

In some embodiments, the sense strand or the antisense strand of the RNAi-inducing nucleic acid molecule may include at least one modification selected from the group consisting of: modification of -OH group at the 2' carbon position of a sugar structure of two or more nucleotides with methoxy (-OCH₃) or fluorine (-F); modification of 10% or more of nucleotide linkages in the sense or antisense strand with phosphorothioate; linkage of cholesterol or palmitic acid to the 3' end of the sense strand; and linkage of a phosphate group to the 5' end of the antisense strand.

Preferably, the sense strand may be any one selected from the group consisting of (a) to (l) of Table 1 below, and the antisense strand may be any one selected from the group consisting of (m) to (x) of Table 1 below.

**[Table 1]**

| | Sequence (5'>3') |
|---|---|
| (a) | mCmCAACUUGUUUACC∗mU∗mA∗chol |
| (b) | mCCmAAmCUmUGmUUmUAmCC∗mU∗A∗chol |
| (c) | mCCmAmACUUmGUUUmACC∗U∗mA∗chol |
| (d) | mCmCAAmCmUmUGmUmUmUAmCmC∗mU∗A∗chol |
| (e) | mCfCmAfAmCfUmUfGmUfUmUfAmCfC∗mU∗fA∗chol |
| (f) | mCmAGAAGCUUCAGGA∗mA∗mA∗chol |
| (g) | mCAmGAmAGmCUmUCmAGmGA∗mA∗A∗chol |
| (h) | mCmAmGmAmAmGCUUCmAmGmGmA∗mA∗mA∗chol |
| (i) | mCAGAAGmCmUmUmCAGGA∗A∗A∗chol |
| (j) | mCmAmGmAmAmGfCfUfUfCmAmGmGmA∗mA∗mA∗chol |
| (k) | mCfAmGfAmAfGmCfUmUfCmAfGmGfA∗mA∗fA∗chol |
| (1) | mCmCAACUUGUUUACC∗mU∗mA∗PA |
| (m) | P-mUAmGGmUAmAAmCAmAGmUUmG∗G∗mG∗U∗mA |
| (n) | P-mUAGGUAAACmAmAmGUUmG∗mG∗mG∗U∗mA |
| (o) | P-mUAGGmUAAAmCAAGmUUG∗G∗G∗mU∗A |
| (p) | P-mUAGGfUAAAfCAAGfUfUG∗G∗G∗fU∗A |
| (q) | P-mUfAmGfGmUfAmAfAmCfAmAfGmUfUmG∗fG∗mG∗fU∗mA |
| (r) | P-mUUmUCmCUmGAmAGmCUmUCmU∗G∗mG∗A∗mC |
| (s) | P-mUUUCCUGAmAmGmCmUmUCmU∗mG∗mG∗mA∗mC |
| (t) | P-mUUUCCUmGmAmAmGCUUCU∗mG∗mG∗mA∗C |
| (u) | P-mUUUCCUGAAGmCmUmUCmU∗G∗G∗A∗mC |
| (v) | P-mUfUfUfCfCfUmGmAmAmGfCfUfUfCfU∗mG∗mG∗mA∗fC |
| (w) | P-mUfUfUfCfCfUGAAGfCfUfUfCfU∗G∗G∗A∗fC |
| (x) | P-mUfUmUfCmCfUmGfAmAfGmCfUmUfCmU∗fG∗mG∗fA∗mC |

In the above sequences, * denotes a phosphorothioated bond, m denotes 2'-O-methyl, 2'-F- denotes 2'-fluoro, chol denotes cholesterol, PA denotes palmitic acid, and P denotes a 5'-phosphate group.

Specifically, the sense strand may be any one of (a) to (e) and (l) of Table 1, and the antisense strand may be any one of (m) to (q) of Table 1. The sense strand may be any one of (f) to (k) of Table 1, and the antisense strand may be any one of (r) to (x) of Table 1. The sense strand may be (a) or (l) of Table 1, and the antisense strand may be (p) or (q) of Table 1.

In the present disclosure, 1 to 3 phosphate group(s) may be bonded to the 5' end of the antisense strand, but the present disclosure is not limited thereto.

In an embodiment of the present disclosure, three phosphate linkages were commonly substituted with phosphorothioate linkages at the 3' end of the sense strand, and cholesterol or palmitic acid was added. Four phosphate linkages were commonly substituted with phosphorothioate linkages at the 3' end of the antisense strand. In addition, 12 sense strands and 12 antisense strands were synthesized by varying the number and position of substitutions of OMethyl and fluoro in the 2' of sugar. A total of 68 cases were annealed to select modifications that best knock down ROR-beta without a carrier, and ROR-beta cp-asiRNA, which knocked down ROR-beta most efficiently, was selected.

Another aspect of the present disclosure relates to a pharmaceutical composition for ameliorating or treating a retinal disease, including the RNAi-inducing nucleic acid molecule.

In the present disclosure, the retinal disease may be Usher syndrome, Stargardt disease, Bardet-Biedl syndrome, Best's disease, choroideremia, gyrate-atrophy, retinitis pigmentosa, retinal macular degeneration, Leber Congenital Amaurosis (Leber's Hereditary Optic Neuropathy), blue-cone monochromacy (BCM), retinoschisis, Malattia Leventinese (ML), Oguchi disease, or Refsum disease, but the present disclosure is not limited thereto.

The pharmaceutical composition may be prepared by including at least one pharmaceutically acceptable carrier in addition to the RNAi-inducing nucleic acid molecule as an active ingredient. The pharmaceutically acceptable carrier is compatible with the active ingredient of the present disclosure and may include saline, sterile water, Ringer's solution, buffered saline, dextrose solution, maltodextrin solution, glycerol, ethanol, or a mixture of two or more of these ingredients. Any other general additives such as antioxidants, buffers, and bacteriostatic agents may be added according to a need. In addition, diluents, dispersants, surfactants, binders, and lubricants may be additionally added to prepare injectable formulations such as aqueous solutions, suspensions, and emulsions. In particular, it is preferable to prepare and provide a formulation in a lyophilized form. For the preparation of the lyophilized formulation, a method commonly known in the art may be used, and a stabilizer for lyophilization may be added.

The method of administering the pharmaceutical composition may be determined by one of ordinary skill in the art on the basis of typical symptoms of a patient and the severity of a disease. In addition, the pharmaceutical composition may be formulated in various forms such as powders, tablets, capsules, liquids, injections, ointments, and syrups, and the pharmaceutical composition may be provided in unit-dose or multi-dose containers, such as sealed ampoules and bottles.

The pharmaceutical composition of the present disclosure may be administered orally or parenterally. The administration route of the composition according to the present disclosure may be, for example, intravitreal injection (IVT), oral administration, intravenous administration, intramuscular administration, intraarterial administration, intramedullary administration, intrathecal administration, intracardiac administration, transdermal administration, subcutaneous administration, intraperitoneal administration, intestinal administration, sublingual administration, or topical administration, but the present disclosure is not limited thereto. The dosage of the composition according to the present invention may vary depending on the body weight and age of a patient, gender, health condition, diet, administration time, administration method, excretion rate, or the severity of a disease, and may be determined by one of ordinary skill in the art. In addition, the composition of the present invention may be formulated into a suitable formulation using known techniques for clinical administration.

Another aspect of the present disclosure relates to a method of ameliorating or treating a retinal disease, including administering the RNAi-inducing nucleic acid molecule to a subject. The configuration included in the method of ameliorating or treating according to the present invention is the same as the configuration included in the disclosure described above, and thus the foregoing description may also equally apply to the method for ameliorating or treating.

The term "administering" may mean placement of a RNAi-inducing nucleic acid molecule according to an embodiment into a subject by a method or pathway that results in at least partial localization of the RNAi-inducing nucleic acid molecule according to one embodiment to a desired site.

The "subject" may be a mammal, for example, a human, a cow, a horse, a pig, a dog, sheep, a goat, a monkey, or a cat. The subject may be an individual in need of ameliorating retinal diseases such as retinitis pigmentosa, for example, suppressing ROR-beta gene expression.

Another aspect of the present disclosure relates to use of the RNAi-inducing nucleic acid molecule for the preparation of a drug for ameliorating or treating a retinal disease. In the above use, the retinal disease, the amelioration, the treatment, and the RNAi-inducing nucleic acid molecule are the same as described above.

Hereinafter, the present disclosure will be described in further detail with reference to the following examples. These examples are only for illustrating the present disclosure, and it will be apparent to those of ordinary skill in the art that the scope of the present disclosure is not construed as being limited by these examples.

### Example 1: 62 Types of RNAi-Inducing Double-Stranded Nucleic Acid Molecules Targeting ROR-beta

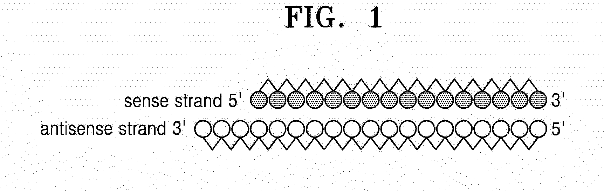

In the present example, ROR-beta asymmetric siRNAs (asiRNAs) for screening were designed. Conventional siRNA is a duplex of 19 base pairs with two nucleotide overhangs at the 3' ends of both strands. asiRNA has a blunt end at the 5' end of an antisense and a short duplex of 15-16 base pairs, thus exhibits inhibitory efficiency equivalent to siRNA and has a reduced off-target effect due to the shortened sense length. Thus, asiRNAs of 16 mer (sense strand)-19 mer (antisense strand) were designed (FIG. 1).

ROR-beta asiRNAs were designed in consideration of the homology of Homo sapiens (Human), Mus musculus (Mouse), Oryctolagus cuniculus (Rabbit), and Macaca mulatta (Monkey) for animal experiments, and the sequences are 100% identical to the corresponding species. Table 2 below shows the sequences of 62 types of ROR-beta asiRNA.

**[Table 2]**

| **No.** | **Name** | **Sequence (5' → 3')** | | Sequence no. |
|---|---|---|---|---|
| 1 | asiRORB-001 | sense | AAAUUUGUGGCGAUAA | 1 |
| | | antisense | UUAUCGCCACAAAUUUUGC | 2 |
| 2 | asiR0RB-002 | sense | AAAAUUUGUGGCGAUA | 3 |
| | | antisense | UAUCGCCACAAAUUUUGCA | 4 |
| 3 | asiRORB-003 | sense | ACCAUGCAAAAUUUGU | 5 |
| | | antisense | ACAAAUUUUGCAUGGUAUC | 6 |
| 4 | asiRORB-004 | sense | GAUACCAUGCAAAAUU | 7 |
| | | antisense | AAUUUUGCAUGGUAUCACU | 8 |
| 5 | asiRORB-005 | sense | UGAUACCAUGCAAAAU | 9 |
| | | antisense | AUUUUGCAUGGUAUCACUU | 10 |
| 6 | asiRORB-006 | sense | GUGAUACCAUGCAAAA | 11 |
| | | antisense | UUUUGCAUGGUAUCACUUC | 12 |
| 7 | as i R0RB-007 | sense | AGUGAUACCAUGCAAA | 13 |
| | | antisense | UUUGCAUGGUAUCACUUCA | 14 |
| 8 | asiRORB-008 | sense | AAGUGAUACCAUGCAA | 15 |
| | | antisense | UUGCAUGGUAUCACUUCAA | 16 |
| 9 | asiR0RB-009 | sense | AUUGAAGUGAUACCAU | 17 |
| | | antisense | AUGGUAUCACUUCAAUUUG | 18 |
| 10 | asiRORB-010 | sense | CACUACGGAGUCAUCA | 19 |
| | | antisense | UGAUGACUCCGUAGUGGAU | 20 |
| 11 | asiRORB-011 | sense | UCCACUACGGAGUCAU | 21 |
| | | antisense | AUGACUCCGUAGUGGAUCC | 22 |
| 12 | asiRORB-012 | sense | AUCCACUACGGAGUCA | 23 |
| | | antisense | UGACUCCGUAGUGGAUCCC | 24 |
| 13 | asiRORB-013 | sense | GAGCCAGCAGAACAAU | 25 |
| | | antisense | AUUGUUCUGCUGGCUCCUC | 26 |
| 14 | asiRORB-014 | sense | GGAGCCAGCAGAACAA | 27 |
| | | antisense | UUGUUCUGCUGGCUCCUCC | 28 |
| 15 | asiRORB-015 | sense | AGGAGCCAGCAGAACA | 29 |
| | | antisense | UGUUCUGCUGGCUCCUCCU | 30 |
| 16 | asiRORB-016 | sense | UGUUUAAUUGACAGAA | 31 |
| | | antisense | UUCUGUCAAUUAAACAGUU | 32 |
| 17 | asiR0RB-017 | sense | CUGUUUAAUUGACAGA | 33 |
| | | antisense | UCUGUCAAUUAAACAGUUU | 34 |
| 18 | asiRORB-018 | sense | CUGAGGUGCAGAAGCA | 35 |
| | | antisense | UGCUUCUGCACCUCAGCAU | 36 |
| 19 | asiRORB-019 | sense | AUGCUGAGGUGCAGAA | 37 |
| | | antisense | UUCUGCACCUCAGCAUACA | 38 |
| 20 | asiR0RB-020 | sense | AGCAGCAGAGUGGGGA | 39 |
| | | antisense | UCCCCACUCUGCUGCUGCC | 40 |
| 21 | asiR0RB-021 | sense | UGUACAGCAGCAGCAU | 41 |
| | | antisense | AUGCUGCUGCUGUACACCC | 42 |
| 22 | asiRORB-022 | sense | AUUGACCUGCCCAAGU | 43 |
| | | antisense | ACUUGGGCAGGUCAAUGAC | 44 |
| 23 | asiRORB-023 | sense | ACGGGCACGUCAUUGA | 45 |
| | | antisense | UCAAUGACGUGCCCGUUGG | 46 |
| 24 | asiR0RB-024 | sense | UUGUUUACCUAUAGCU | 47 |
| | | antisense | AGCUAUAGGUAAACAAGUU | 48 |
| 25 | asiR0RB-025 | sense | CAACUUGUUUACCUAU | 49 |
| | | antisense | AUAGGUAAACAAGUCGGGU | 50 |
| 26 | asiRORB-026 | sense | CCAACUUGUUUACCUA | 51 |
| | | antisense | UAGGUAAACAAGUUGGGUA | 52 |
| 27 | asiRORB-027 | sense | UUGCACAGAACAUCAU | 53 |
| | | antisense | AUGAUGUUCUGUGCAAUUC | 54 |
| 28 | asiRORB-028 | sense | AUUGCACAGAACAUCA | 55 |
| | | antisense | UGAUGUUCUGUGCAAUUCG | 56 |
| 29 | asiRORB-029 | sense | AAAGCAAGUCCAGGGA | 57 |
| | | antisense | UCCCUGGACUUGCUUUGAU | 58 |
| 30 | asiRORB-030 | sense | UAACAGGCUUCAUGGA | 59 |
| | | antisense | UCCAUGAAGCCUGUCAUCC | 60 |
| 31 | asiRORB-031 | sense | GGAUAACAGGCUUCAU | 61 |
| | | antisense | AUGAAGCCUGUUAUCCGCU | 62 |
| 32 | asiRORB-032 | sense | CGGAUAACAGGCUUCA | 63 |
| | | antisense | UGAAGCCUGUUAUCCGCUU | 64 |
| 33 | asiRORB-033 | sense | GAAAAUAUGGAGGAAU | 65 |
| | | antisense | AUUCCUCCAUAUUUUCCUU | 66 |
| 34 | asiRORB-034 | sense | GGAAAAUAUGGAGGAA | 67 |
| | | antisense | UUCCUCCAUAUUUUCCUUC | 68 |
| 35 | asiRORB-035 | sense | UGAAGGAAAAUAUGGA | 69 |
| | | antisense | UCCAUAUUUUCCUUCAAAC | 70 |
| 36 | asiR0RB-036 | sense | CUGUUUGAAGGAAAAU | 71 |
| | | antisense | AUUUUCCUUCAAACAGAAC | 72 |
| 37 | asiRORB-037 | sense | UCUGUUUGAAGGAAAA | 73 |
| | | antisense | UUUUCCUUCAAACAGAACA | 74 |
| 38 | asiRORB-038 | sense | UUCUGUUUGAAGGAAA | 75 |
| | | antisense | UUUCCUUCAAACAGAACAG | 76 |
| 39 | asiRORB-039 | sense | GUUCUGUUUGAAGGAA | 77 |
| | | antisense | UUCCUUCAAACAGAACAGU | 78 |
| 40 | asiRORB-040 | sense | CACUGUUCUGUUUGAA | 79 |
| | | antisense | UUCAAACAGAACAGUGUUG | 80 |
| 41 | asiR0RB-041 | sense | ACACUGUUCUGUUUGA | 81 |
| | | antisense | UCAAACAGAACAGUGUUGU | 82 |
| 42 | asiR0RB-042 | sense | CAACACUGUUCUGUUU | 83 |
| | | antisense | AAACAGAACAGUGUUGUUU | 84 |
| 43 | asiRORB-043 | sense | ACAACACUGUUCUGUU | 85 |
| | | antisense | AACAGAACAGUGUUGUUUA | 86 |
| 44 | asiRORB-044 | sense | UAAACAACACUGUUCU | 87 |
| | | antisense | AGAACAGUGUUGUUUAAUG | 88 |
| 45 | asiR()RB-045 | sense | UUCAAAGCCUUAGGUU | 89 |
| | | antisense | AACCUAAGGCUUUGAACAU | 90 |
| 46 | asiRORB-046 | sense | UGAAGCAUUUGACUUU | 91 |
| | | antisense | AAAGUCAAAUGCUUCAUUC | 92 |
| 47 | asiRORB-047 | sense | AUGAAGCAUUUGACUU | 93 |
| | | antisense | AAGUCAAAUGCUUCAUUCA | 94 |
| 48 | asiRORB-048 | sense | AAUGAAGCAUUUGACU | 95 |
| | | antisense | AGUCAAAUGCUUCAUUCAC | 96 |
| 49 | as i R0RB-049 | sense | UGAAUGAAGCAUUUGA | 97 |
| | | antisense | UCAAAUGCUUCAUUCACUA | 98 |
| 50 | asiRORB-050 | sense | AGUGAAUGAAGCAUUU | 99 |
| | | antisense | AAAUGCUUCAUUCACUAGG | 100 |
| 51 | asiRORB-051 | sense | UAGUGAAUGAAGCAUU | 102 |
| | | antisense | AAUGCUUCAUUCACUAGGU | 102 |
| 52 | asiRORB-052 | sense | CUAGUGAAUGAAGCAU | 103 |
| | | antisense | AUGCUUCAUUCACUAGGUC | 104 |
| 53 | asiRORB-053 | sense | GUUCCUCGCAGCUGAC | 105 |
| | | antisense | GUCAGCUGCAAGGAACACA | 106 |
| 54 | asiRORB-054 | sense | AGAAGCUUCAGGAAAA | 107 |
| | | antisense | UUUUCCUGAAGCUUCUGGA | 108 |
| 55 | asiR0RB-055 | sense | CAGAAGCUUCAGGAAA | 109 |
| | | antisense | UUUCCUGAAGCUUCUGGAC | 110 |
| 56 | asiRORB-056 | sense | AUGUGAUECAGAAGAA | 111 |
| | | antisense | UUCUUCCGAAUCACAUGUU | 112 |
| 57 | asiRORB-057 | sense | CAUGUGAUUCAGAAGA | 113 |
| | | antisense | UCUUCUGAAUCACAUGUUG | 114 |
| 58 | asiRORB-058 | sense | AUAGCCAAGAUACCAA | 115 |
| | | antisense | UUGGUAUCUUGGCUAUUAA | 116 |
| 59 | asiRORB-059 | sense | GUUAAUAGCCAAGAUA | 117 |
| | | antisense | UAUCUUGGCUAUUAACUUU | 118 |
| 60 | asiRORB-060 | sense | AGUUAALAGCCAAGAC | 119 |
| | | antisense | AUCUUGGCUAUUAACUUUG | 120 |
| 61 | asiRORB-061 | sense | AAGUUAAUAGCCAAGA | 121 |
| | | antisense | UCUUGGCUAUUAACUUUGC | 122 |
| 62 | asiRORB-062 | sense | GAAUACACUGUUUCCU | 123 |
| | | antisense | AGGAAACAGUGUAUUCACU | 124 |

### Example 2: Screening of RNAi-inducing Double-Stranded Nucleic Acid Molecules Targeting ROR-beta

In this example, to confirm the expression inhibitory effects of the ROR-beta asiRNAs of Example 1, Y-79 cells (ATCC) were transfected with a 10 nM asymmetric siRNA, and then the expression level of the ROR-beta protein was analyzed through Western blotting. Specifically, Y-79 cells were seeded in a 12-well plate at a density of 1×10⁵ cells/well, and then transfected with 10 nM asiRNA using Lipofectamine RNAiMax Transfection Reagent (Invitrogen, 13778030) for 48 hours according to the protocol provided by Invitrogen. Thereafter, the transfected cells were disrupted to obtain a cell lysate, followed by western blotting. For the obtained cell lysate, 12% SDS-polyacrylamide gel, an ROR-beta antibody (Proteintech, Cat. #17635-1), and a Vinculin antibody (Santa Cruz Biotechonology, Cat. # sc-73614) were used to measure the expression level of the ROR-beta protein according to the protocol of each manufacturer. In this example, an untreated group was used as a negative control.

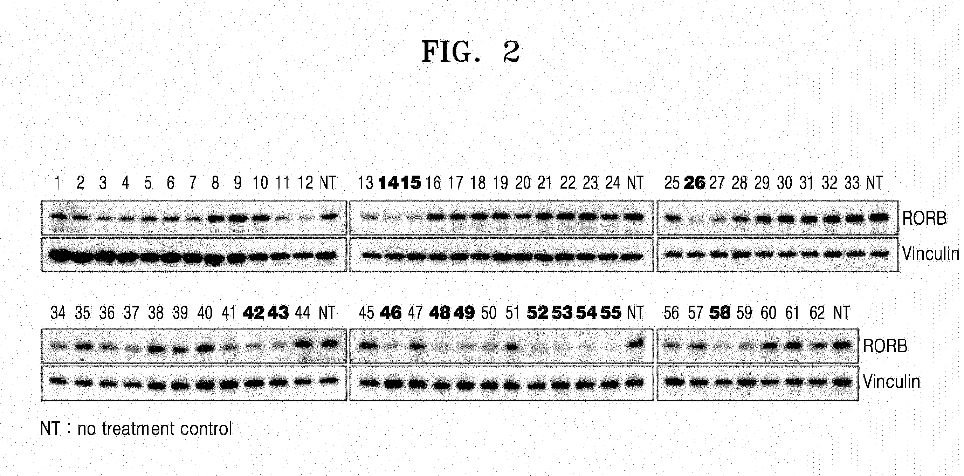

As a result, as illustrated in FIG. 2, the inhibitory effect of ROR-beta protein expression according to treatment with ROR-beta asiRNA was confirmed. Specifically, among 62 types of ROR-beta asiRNA, 13 types of asymmetric siRNA exhibiting excellent ROR-beta protein expression inhibitory effects (#14, #15, #26, #42, #43, #46, #48, #49, #52, #53, #54, #55, #58) were identified.

### Example 3: Confirmation of ROR-Beta Expression Inhibitory Efficiency for Obtained asiRNAs

### 3.1. Confirmation of ROR-beta Protein Expression Inhibitory Effect

In this example, to confirm a protein expression inhibitory effect by the ROR-beta asiRNAs obtained in Example 2 (#14, #15, #26, #42, #43, #46, #48, #49, #52, #53, #54, # 55, and #58), Y-79 cells (ATCC) were transfected with 1 nM or 5 nM of each asymmetric siRNA, and then the expression level of the ROR-beta protein was measured through western blotting. Specifically, Y-79 cells were seeded into a 12-well plate at a density of 1 × 10⁵ cells/well, and then were transfected with 1 nM or 5 nM of each asiRNA using Lipofectamine RNAiMax Transfection Reagent (Invitrogen, 13778030) for 48 hours according to the protocol provided by Invitrogen. Thereafter, the transfected cells were disrupted to obtain a cell lysate, followed by western blotting. In the same manner as in Example 2, for the obtained cell lysate, 12% SDS-polyacrylamide gel, an ROR-beta antibody (Proteintech, Cat. #17635-1), and a Vinculin antibody (Santa Cruz Biotechonology, Cat. # sc-73614) were used to measure the expression level of the ROR-beta protein according to the protocol of each manufacturer. In this example, an untreated group was used as a negative control.

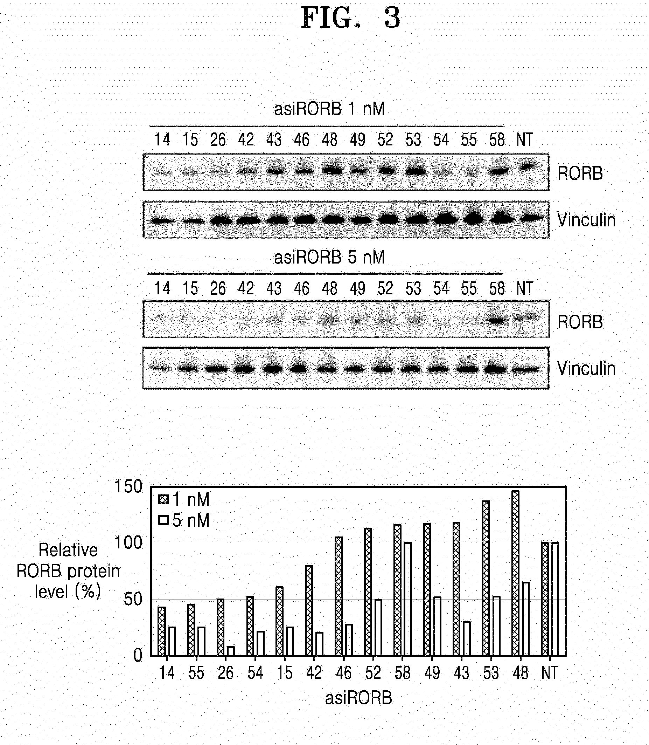

As a result, as illustrated in FIG. 3, ROR-beta protein expression inhibitory effects in a concentration-dependent manner according to treatment with the ROR-beta asiRNAs were confirmed. In particular, on the basis of the results of 1 nM treatment, about 50% or more of protein expression inhibition was observed in #14, #26, and #55.

### 3.2. Confirmation of ROR-beta mRNA Expression Inhibitory Effect

In this example, to confirm mRNA expression inhibitory effects by the ROR-beta asiRNAs obtained in Example 2 (#14, #15, #26, #42, #43, #46, #48, #49, #52, #53, #54, # 55, and #58), Y-79 cells (ATCC) were transfected with 1 nM or 10 nM of each asymmetric siRNA, and then the expression level of ROR-beta mRNA was measured by real-time PCR. Specifically, Y-79 cells were seeded into a 24-well plate at a density of 6 × 10⁴ cells/well, and then were transfected with 1 nM or 10 nM of each asiRNA using Lipofectamine RNAiMax Transfection Reagent (Invitrogen, 13778030) for 24 hours according to the protocol provided by Invitrogen. Then, total RNA was extracted using Tri-RNA reagent (FAVORGEN, FATRR001), and then cDNA was synthesized using a High-capacity cDNA reverse transcription kit (Applied Biosystems, 4368813). Thereafter, ROR-beta (Hs00199445_m1) and RN18S1 (Hs03928985_g1) of TaqMan^{®} Gene Expression Assays (Applied Biosystems) and THUNDERBIRD^{®} Probe qPCR Mix (TOYOBO, QPS-101) were used to confirm the expression level of ROR-beta mRNA by CFX Connect Real-Time PCR Detection System (BioRad). The expression level of ROR-beta mRNA was normalized using RN18S1, and an untreated group was used as a negative control.

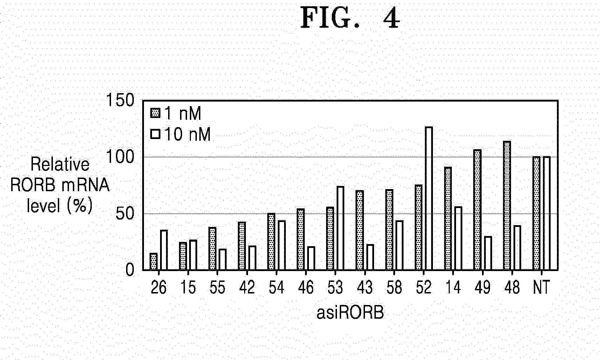

As illustrated in FIG. 4, the ROR-beta mRNA expression inhibitory effect according to ROR-beta asiRNA was confirmed. In particular, about 60% or more of mRNA expression inhibition was observed in #15, #26, and #55 on the basis of the results of 1 nM treatment.

Through the results of Example 3, two types (#26 and #55) included in all of the top three sequences with excellent ROR-beta mRNA and protein expression inhibitory effects were obtained.

### Example 4: Design and Production of Chemical Modification-Introduced Asymmetric siRNAs

In this example, for the two types (#26 and #55) from among the ROR-beta asiRNAs of which expression inhibitory effects were confirmed in Example 3, chemical modification-introduced asymmetric siRNAs (cell penetrating-asymmetric siRNAs: cp-asiRNAs) were designed. Compared to the asiRNAs, the cp-asiRNAs designed in this example are asymmetric siRNAs that have been subjected to various chemical modifications (2'OMe, PS, and Fluoro) and have improved delivery into cells.

Sequence information of a total of 67 cp-asiRNAs produced in this example is shown in Table 3 below.

**[Table 3]**

| **No.** | **Name** | **Sequence (5' → 3')** | |
|---|---|---|---|
| 1 | 0LX304C-026-1 | sense | mCmCAACUUGUUUACC∗mU∗mA∗chol |
| | | antisense | P-mUAmGGmUAmAAmCAmAGmUUmG∗G∗mG∗U∗mA |
| 2 | 0LX304C-026-2 | sense | mCmCAACUUGUUUACC∗mU∗mA∗chol |
| | | antisense | P-mUAGGUAAACmAmAmGUUmG∗mG∗mG∗U∗mA |
| 3 | 0LX304C-026-3 | sense | mCmCAACUUGUUUACC∗mU∗mA∗chol |
| | | antisense | P-mUAGGmUAAAmCAAGmUUG∗G∗G∗mU∗A |
| 4 | 0LX304C-026-4 | sense | mCmCAACUUGUUUACC∗mU∗mA∗chol |
| | | antisense | P-mUAGGfUAAAfCAAGfUfUG∗G∗G∗fU∗A |
| 5 | 0LX304C-026-5 | sense | mCmCAACUUGUUUACC∗mU∗mA∗chol |
| | | antisense | P-mUfAmGfGmUfAmAfAmCfAmAfGmUfUmG∗fG∗mG∗fU∗mA |
| 6 | 0LX304C-026-6 | sense | mCCmAAmCUmUGmUUmUAmCC∗mU∗A∗chol |
| | | antisense | P-mUAmGGmUAmAAmCAmAGmUUmG∗G∗mG∗U∗mA |
| 7 | 0LX304C-026-7 | sense | mCCmAAmCUmUGmUUmUAmCC∗mU∗A∗chol |
| | | antisense | P-mUAGGUAAACmAmAmGUUmG∗mG∗mG∗U∗mA |
| 8 | 0LX304C-026-8 | sense | mCCmAAmCUmUGmUUmUAmCC∗mU∗A∗chol |
| | | antisense | P-mUAGGmUAAAmCAAGmUUG∗G∗G∗mU∗A |
| 9 | 0LX304C-026-9 | sense | mCCmAAmCUmUGmUUmUAmCC∗mU∗A∗chol |
| | | antisense | P-mUAGGfUAAAfCAAGfUfUG∗G∗G∗fU∗A |
| 10 | 0LX304C-026-10 | sense | mCCmAAmCUmUGmUUmUAmCC∗mU∗A∗chol |
| | | antisense | P-mUfAmGfGmlfAmAf AmCfAmAfGmUfUmG*fG*niG*fU*inA |
| 11 | 0LX304C-026-11 | sense | mCCmAmACUUmGUUUmACC∗U∗mA∗chol |
| | | antisense | P-mUAmGGmUAmAAmCAmAGmUUmG∗G∗mG∗U∗mA |
| 12 | 0LX304C-026-12 | sense | mCCmAmACUUmGUUUmACC∗U∗mA∗chol |
| | | antisense | P-mUAGGUAAACmAmAmGUUmG∗mG∗mG∗U∗mA |
| 13 | 0LX304C-026-13 | sense | mCCmAmACUUmGUUUmACC∗U∗mA∗chol |
| | | antisense | P-mUAGGmUAAAmCAAGmUUG∗G∗G∗mU∗A |
| 14 | 0LX304C-026-14 | sense | mCCmAmACUUmGUUUmACC∗U∗mA∗chol |
| | | antisense | P-mUAGGfUAAAfCAAGfUfUG∗G∗G∗fU∗A |
| 15 | 0LX304C-026-15 | sense | mCCmAmACUUmGUUUmACC∗U∗mA∗chol |
| | | antisense | P-mUfAmGfGmUfAmAfAmCfAmAfGmUfUmG∗fU∗mA |
| 16 | OLX304C-026-16 | sense | mCmCAAmCmUmUGmUmUmUAmCmC∗mU∗A∗chol |
| | | antisense | P-mUAmGGmUAmAAmCAmAGmUUmG∗G∗mG∗U∗mA |
| 17 | ULX304C-026-17 | sense | mCmCAAmCmUmUGmUmUmUAmCmC∗mU∗A∗chol |
| | | antisense | P-mUAGGUAAACmAmAmGUUmG∗mG∗mG∗U∗mA |
| 18 | OLX304C-026-18 | sense | mCmCAAmCmUmUGmUmUmUAmCmC∗mU∗A∗chol |
| | | antisense | P-mUAGGmUAAAmCAAGmUUG∗G∗G∗mU∗A |
| 19 | OLX304C-026-19 | sense | mCmCAAmCmUmUGmUmUmUAmCmC*mU∗A∗chol |
| | | antisense | P-mUAGGfUAAAfCAAGfUfUG∗G∗G∗fU∗A |
| 20 | OLX304C-026-20 | sense | mCmCAAmCmUmUGmUmUmUAmCmC∗mU∗A∗chol |
| | | antisense | P-mUfAmGfGmUfAmAfAmCfAmAfGmUfUmG∗fG∗mG∗fU∗mA |
| 21 | OLX304C-026-21 | sense | mCfCmAfAmCfUmUfGmUfUmUfAmCfC∗mU∗fA∗chol |
| | | antisense | P-mUAmGGmUAmAAmCAmAGmUUmG∗G∗mG∗U∗mA |
| 22 | OLX304C-026-22 | sense | mCfCmAfAmCfUmUfGmUfUmUfAmCfC∗mU∗fA∗chol |
| | | antisense | P-mUAGGUAAACmAmAmGUUmG∗mG∗mG∗U∗mA |
| 23 | OLX304C-026-23 | sense | mCfCmAfAmCfUmUfGmUfGmUfUmUfAmCfC∗fA∗chol |
| | | antisense | P-mUAGGmUAAAmCAAGmUUG∗G∗G∗mU∗A |
| 24 | OLX304C-026-24 | sense | mCfCmAfAmCfUmUfGmUfUfAmCfC∗mU∗fA∗chol |
| | | antisense | P-mUAGGfUAAAfCAAGfUfUG∗G∗G∗fU∗A |
| 25 | OLX304C-026-25 | sense | mCfCmAfAmCfUmUfGmUfUmUfAmCfC∗mU∗fA∗chol |
| | | antisense | P-mUfAmGfGmUfAmAfAmCfAmAfGmUfUmG∗fG∗mG∗fU∗mA |
| 26 | OLX304C-055-1 | sense | mCmAGAAGCUUCAGGA∗mA∗mA∗chol |
| | | ant i sense | P-mUUmUCmCUmGAmAGmCUmUCmU∗G∗mG∗A∗mC |
| 27 | OLX304C-055-2 | sense | mCmAGAAGCUUCAGGA∗mA∗mA∗chol |
| | | antisense | P-mUUUCCUGAmAmGmCmUmUCmU∗mG∗mG∗mA∗mC |
| 28 | OLX304C-055-3 | sense | mCmAGAAGCUUCAGGA∗mA∗mA∗chol |
| | | ant i sense | P-mUUUCCUmGmAmAmGCUUCU∗mG∗mG∗mA∗C |
| 29 | OLX304C-055-4 | sense | mCmAGAAGCUUCAGGA∗mA∗mA∗chol |
| | | antisense | P-mUUUCCUGAAGmCmUmUCmU∗G∗G∗A∗mC |
| 30 | OLX304C-055-5 | sense | mCmAGAAGCUUCAGGA∗mA∗mA∗chol |
| | | antisense | P-mUfUfUfCfCfUmGmAmAmGfCfUfUfCfU∗mG∗mG∗mA∗fC |
| 31 | OLX304C-055-6 | sense | mCmAGAAGCUUCAGGA∗mA∗mA∗chol |
| | | antisense | P-mUfUfUfCfCfUGAAGfCfUfUfCfU∗G∗G∗A∗fC |
| 32 | OLX304C-055-7 | sense | mCmAGAAGCUUCAGGA∗mA∗mA∗chol |
| | | antisense | P-mUfUmUfCmCfUmGfAmAfGmCfUmUfCmU∗fG∗mG∗fA∗mC |
| 33 | OLX304C-055-8 | sense | mCAmGAmAGmCUmUCmAGmGA∗mA∗A∗chol |
| | | antisense | P-mUUmUCmCUmGAmAGmCUmUCmU∗G∗mG∗A∗mC |
| 34 | OLX304C-055-9 | sense | mCAmGAmAGmCUmUCmAGmGA∗mA∗A∗chol |
| | | antisense | P-mUUUCCUG.AmAmGmCmUmUCmU∗mG∗mG∗mA∗mC |
| 35 | OLX304C-055-10 | sense | mCAmGAmAGmCUmUCmAGmGA∗mA∗A∗chol |
| | | antisense | P-mUUUCCUmGmAmAmGCUUCU∗mG∗mG∗mA∗C |
| 36 | OLX304C-055-11 | sense | mCAmGAmAGmCUmUCmAGmGA∗mA∗A∗chol |
| | | antisense | P-mUUUCCUGAAGmCmUmUCmU∗G∗G∗A∗mC |
| 37 | OLX304C-055-12 | sense | mCAmGAmAGmCUmUCmAGmGA∗mA∗A∗chol |
| | | ant i sense | P-mUfUfUfCfCfUmGmAmAmGfCfUfUfCfU∗mG∗mG∗mA∗fC |
| 38 | OLX304C-055-13 | sense | mCAmGAmAGmCUmUCmAGmGA∗mA∗A∗chol |
| | | antisense | P-mUfUfUfCfCfUGAAGfCfUfUfUfCfU∗G∗G∗A∗fC |
| 39 | OLX304C-055-14 | sense | mCAmGAmAGmCUmUCmAGmGA∗mA∗A∗chol |
| | | antisense | P-mUfUmUfCmCfUmGfAmAfGmCfUmUfCmU∗fG∗mG∗fA∗mC |
| 40 | OLX304C-055-15 | sense | mCmAmGmAmAmGCUUCmAmGmGmA∗mA∗mA∗chol |
| | | antisense | P-mUUmUCmCUmGAmAGmCUmUCmU∗G∗mG∗A∗mC |
| 41 | OLX304C-055-16 | sense | mCmAmGmAmAmGCUUCmAmGmGmA∗mA∗mA∗chol |
| | | antisense | P-mUUUCCUGAmAmGmCmUmUCmU∗mG∗mA∗mC |
| 42 | OLX304C-055-17 | sense | mCmAmGmAmAmGCCUCmAmGmGmA∗mA∗mA∗chol |
| | | antisense | P-mUUUCCUmGmAmAmGCUUCU∗mG∗mG∗mA∗C |
| 43 | OLX304C-055-18 | sense | mCmAmGmAmAmGCCUCmAmGmGmA∗mA∗mA∗chol |
| | | antisense | P-mUUUCCCUGAAGmCmUmUCmU∗G∗G∗A∗mC |
| 44 | OLX304C-055-19 | sense | mCmAmGmAmAmGCUUCmAmGmGmA∗mA∗mA∗chol |
| | | antisense | P-mUfUfUfCfCfUmGmAmAmGfCfUfUfCfU∗mG∗mG∗mA∗fC |
| 45 | OLX304C-055-20 | sense | mCmAmGmAmAmGCUUCmAmGmGmA∗mA∗mA∗chol |
| | | antisense | P-mUfUfUfCfUGAAGfCfUfUfCfU∗G∗G∗A∗fC |
| 46 | OLX304C-055-21 | sense | mCmAmGmAmAmGCUUCmAmGmGmA∗mA∗mA∗chol |
| | | antisense | P-mUfUmUfCmCfUmGfAmAfGmCfUmUfCmU∗fG∗mG∗fA∗mC |
| 47 | OLX304C-055-22 | sense | mCAGAAGmCmUmUmCAGGA∗A∗A∗chol |
| | | antisense | P-mUUmUCmCUmGAmAGmCUmUCmU∗G∗mG∗A∗mC |
| 48 | 0LX304C-055-23 | sense | mCAGAAGmCmUmUmCAGGA∗A∗A∗chol |
| | | antisense | P-mUUUCCUGAmAmGmCmUmUCmU∗mG∗mG∗mA∗mC |
| 49 | OLX304C-055-24 | sense | mCAGAAGmCmUmUmCAGGA∗A∗A∗chol |
| | | antisense | P-mUUUCCUmGmAmAmGCUUCU∗mG∗mG∗mA∗C |
| 50 | OLX304C-055-25 | sense | mCAGAAGmCmUmUmCAGGA∗A∗A∗chol |
| | | antisense | P-mUUUCCUGAAGmCmUmUCmU∗G∗G∗A∗mC |
| 51 | 01,X304C-055-26 | sense | mCAGAAGmCmUmUmCAGGA∗A∗A∗chol |
| | | antisense | P-mUfUfUfCfCfUmGmAmAmGfCfUfUfCfU∗mG∗mG∗mA∗fC |
| 52 | 0LX304C-055-27 | sense | mCAGAAGmCmUmUmCAGGA∗A∗A∗chol |
| | | antisense | P-mUfUfUfCfCfUGAAGfCfUfUfCfU∗G∗G∗A∗fC |
| 53 | OLX304C-055-28 | sense | mCAGAAGmCmUmUmCAGGA∗A∗A∗chol |
| | | antisense | P-mUfUmUfCmCfUmGfAmAfGmCfUmUfCmU∗fG∗mG∗fA∗mC |
| 54 | 0LX304C-055-29 | sense | mCmAmGmAmAmGfCfUfUfCmAmGmGmA∗mA∗mA∗chol |
| | | antisense | P-mUUmUCmCUmGAmAGmCUmUCmU∗G∗mG∗A∗mC |
| 55 | 0LX304C-055-30 | sense | mCmAmGmAmAmGfCfUfUfCmAmGmGmA∗mA∗mA∗chol |
| | | antisense | P-mUUUCCUGAmAmGmCmUmUCmU∗mG∗mG∗mA∗mC |
| 56 | OLX304C-055-31 | sense | mCmAmGmAmAmGfCfUfUfCmAmGmGmA∗mA∗mA∗chol |
| | | antisense | P-mUUUCCUmGmAmAmGCUUCU∗mG∗mG∗mA∗C |
| 57 | 0LX304C-055-32 | sense | mCmAmGmAmAmGfCfUfUfCmAmGmGmA∗mA∗mA∗chol |
| | | antisense | P-mUUUCCUGAAGmCmUmUCmU∗G∗G∗A∗mC |
| 58 | OLX304C-055-33 | sense | mCmAmGmAmAmGfCfUfUfCmAmGmGmA∗mA∗mA∗chol |
| | | antisense | P-mUfUfUfCfCfUmGmAmAmGfCfUfUfCfU∗mG∗mG∗mA∗fC |
| 59 | 0LX304C-055-34 | sense | mCmAmGmAmAmGfCfUfUfCmAmGmGmA∗mA∗mA∗chol |
| | | antisense | P-mUfUfUfCfCfUGAAGfCfUfUfCfU∗G∗G∗A∗fC |
| 60 | OLX304C-055-35 | sense | mCmAmGmAmAmGfCfUfUfCmAmGmGmA∗mA∗mA∗chol |
| | | antisense | P-mUfUmUfCmCfUmGfAmAfGmCfUmUfCmU∗fG∗mG∗fA∗mC |
| 61 | 0LX304C-055-36 | sense | mCfAmGfAmAfGmCfUmUfCmAfGmGfA∗mA∗fA∗chol |
| | | antisense | P-mUUmUCmCUmGAmAGmCUmUCmU∗G∗mG∗A∗mC |
| 62 | 01,X304C-055-37 | sense | mCfAmGfAmAfGmCfUmUfCmAfGmGfA∗mA∗fA∗chol |
| | | antisense | P-mUUUCCUGAmAmGmCmUmUCmU∗mG∗mG∗mA∗mC |
| 63 | 01,X304C-055-38 | sense | mCfAmGfAmAfGmCfUmUfCmAfGmGfA∗mA∗fA∗chol |
| | | antisense | P-mUUUCCUmGmAmAmGCUUCU∗mG∗mG∗mA∗C |
| 64 | 0LX304C-055-39 | sense | mCfAmGfAmAfGmCfUmUfCmAfGmGfA∗mA∗fA∗chol |
| | | antisense | P-mUUUCCUGAAGmCmUmUCmU∗G∗G∗A∗mC |
| 65 | 0LX304C-055-40 | sense | mCfAmGfAmAfGmCfUmUfCmAfGmGfA∗mA∗fA∗chol |
| | | antisense | P-mUfUfUfCfCfUmGmAmAmGfCfUfUfCfU∗mG∗mG∗mA∗fC |
| 66 | 0LX304C-055-41 | sense | mCfAmGfAmAfGmCfUmUfCmAfGmGfA∗mA∗fA∗chol |
| | | antisense | P-mUfUfUfCfCfUGAAGfCfUfUfCfU∗G∗G∗A∗fC |
| 67 | 0LX304C-055-42 | sense | mCfAmGfAmAfGmCfUmUfCmAfGmGfA∗mA∗fA∗chol |
| | | antisense | P-mUfUmUfCmCfUmGfAmAfGmCfUmUfCmU∗fG∗mG∗fA∗mC |

In Table 3, chemical modifications indicated by "*", "m", "f", and "chol" are the same as shown in Table 4 below.

**[Table 4]**

| **Notation** | **Chemical modification** |
|---|---|
| ^{∗} | **phosphorothioate bond** |
| m | **2'-O-methyl** |
| f | **2'-fluoro** |
| chol | **cholesterol** |

Specifically, in Table 3, "*" denotes a form in which an existing phosphodiester linkage is substituted with a phosphorothioate linkage, and "m" denotes a form in which an existing 2'-OH is substituted with 2'-O-methyl. In addition, "f" denotes, for example, in the case of fG, a form in which 2'-OH of existing guanine (G) is substituted with fluoro, and "Chol" denotes a form in which cholesterol is added to the 3'-end.

### Example 5: Screening of Chemical Modification-Introduced Asymmetric siRNAs

### 5.1 Confirmation of ROR-beta Protein Expression Inhibitory Effect

In this example, to confirm the expression inhibitory effects of the ROR-beta cp-asiRNAs of Example 4, Y-79 cells were treated with each cp-asiRNA and incubated (free uptake), followed by western blotting to measure the expression level of the ROR-beta protein. Specifically, Y-79 cells were seeded into a 12-well plate at a density of 1 × 10⁵ cells/well, and 24 hours later, 1 µM of cp-asiRNA was added thereto, and then the cells were incubated under conditions of RPMI (Gibco, 11875-093) media containing 10% fetal bovine serum (FBS) (Gibco, 16000-044). After 48 hours, the cells were disrupted to obtain a cell lysate, followed by western blotting. For the obtained cell lysate, 12% SDS-polyacrylamide gel, an ROR-beta antibody (Proteintech, Cat. #17635-1), and a Vinculin antibody (Santa Cruz Biotechonology, Cat. # sc-73614) were used to measure the expression level of the ROR-beta protein. In this example, an untreated group was used as a negative control, and a group transfected with ROR-beta asiRNA, which was not chemically modified, at a concentration of 10 nM using lipofectamine RNAiMAX was used as a positive control.

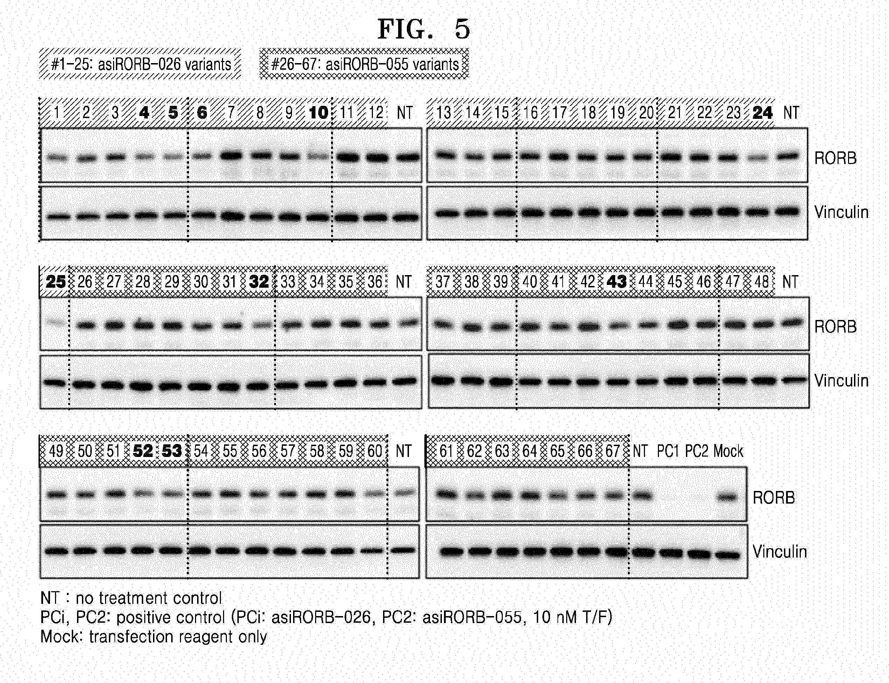

As a result, as illustrated in FIG. 5, the ROR-beta protein expression inhibitory effect according to treatment with ROR-beta cp-asiRNA was confirmed. In particular, among these, 10 types (4, 5, 6, 10, 24, 25, 32, 43, 52, and 53) of ROR-beta cp-asiRNA exhibiting excellent ROR-beta protein expression inhibitory effects were identified.

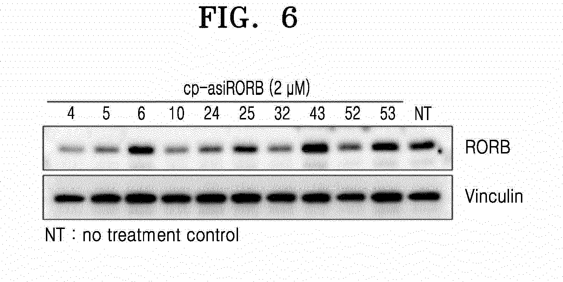

To reconfirm the ROR-beta protein expression inhibitory effects by 10 types of cp-asiRNA, the expression level of the ROR-beta protein was measured after treatment at a concentration of 2 µM in the same manner. As a result, as illustrated in FIG. 6, the ROR-beta protein expression inhibitory effect according to treatment with ROR-beta cp-asiRNA was confirmed. In particular, among these, reproducibility of the ROR-beta protein expression inhibitory effect was confirmed in cp-asiRNAs 4, 5, 10, 24, 25, 32, and 52.

### 5.2. Confirmation of ROR-beta Protein Expression Inhibitory Effect Under ROR-beta Overexpression Conditions

In this example, the protein inhibitory effect of cp-asiRNA was confirmed in cells (transient cell line) transiently expressing ROR-beta by using a ROR-beta plasmid (Origene, RC208666). More specifically, A549 cells were seeded into a 12-well plate at a density of 5 × 10⁴ cells/well using Ham's F-12K (Kaighn's) Medium (Gibco, 21127022) supplemented with 10% FBS, and after 24 hours, 200 ng of a ROR- Beta plasmid was incubated in Lipofectamine 2000 (Invitrogen, 11668-019) and Opti-MEM reduced serum media (gibco, 31985-070), and then the cells were treated therewith. After 6 hours, plate wash was performed using PBS, followed by incubation (free uptake) with 1 µM cp-asiRNA for 24 hours in Opti-MEM reduced serum media. The next day, the medium was replaced with Ham's F-12K (Kaighn's) Medium (10% FBS), and one day later, the cells were disrupted to obtain a cell lysate. Then, 12% SDS-polyacrylamide gel, a ROR-beta antibody (Proteintech, Cat. #17635-1), a Vinculin antibody (Santa Cruz Biotechonology, Cat. #sc-73614), and a Neomycin Phosphotransferase II antibody (Invitrogen, Cat. MA5-15275) were used to measure the expression level of the ROR-beta protein. In this example, Neomycin Phosphotransferase II antibody was used as a control for plasmid transfection, and a ROR-beta plasmid-treated group that was not treated with cp-asiRNA was used as a negative control.

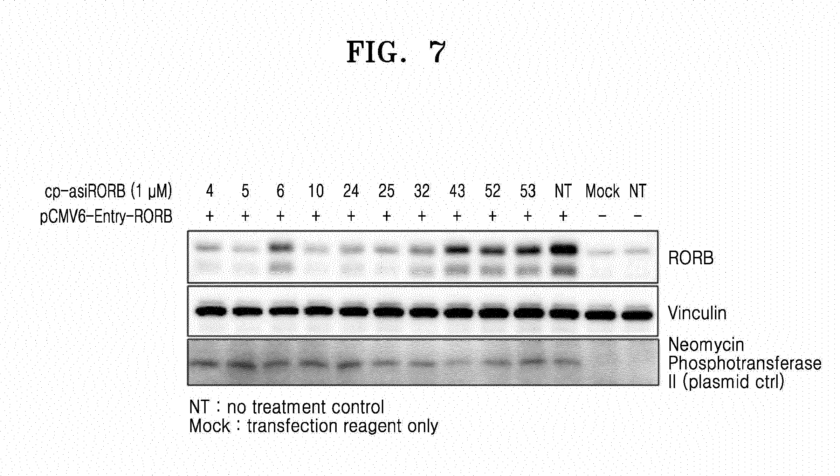

As a result, as illustrated in FIG. 7, a high protein expression inhibitory effect was confirmed compared to the untreated group. In particular, it was confirmed that the results of this example showed a tendency similar to the ROR-beta protein inhibitory effect in a Y-79 cell line of Example 5.1, and through the results of Examples 5.1 and 5.2, 5 types of cp-asiRNA (4, 5, 10, 24, and 25) with excellent ROR-beta protein expression inhibitory effects were identified.

### Example 6: Confirmation of ROR-beta Expression Inhibitory Efficiency for Derived cp-asiRNAs

### 6.1. Confirmation of ROR-beta mRNA Expression Inhibitory Effect

In this example, to confirm the ROR-beta mRNA expression inhibitory effects of the 5 types of ROR-beta cp-asiRNA (4, 5, 10, 24, and 25) derived in Example 5, an A549 cell line transiently expressing ROR-beta was treated with 200 nM or 500 nM of each cp-asiRNA, followed by real-time PCR to measure the expression level of ROR-beta mRNA. Specifically, A549 cells were seeded into a 96-well plate at a density of 8 × 10³ cells/well, and the next day, 10 ng of a ROR-beta plasmid was incubated in Lipofectamine 2000 (Invitrogen, 11668-019) and Opti-MEM reduced serum media (gibco, 31985- 070), and then the cells were treated therewith. After 6 hours, plate wash was performed using PBS, followed by incubation (free uptake) with 200 nM or 500 nM cp-asiRNA for 24 hours in Opti-MEM reduced serum media. Thereafter, cDNA was synthesized from the cell lysate using SuperPrep II Cell lysis & RT Kit for qPCR (TOYOBO, SCQ-101) according to the manufacturer's protocol, and then the expression level of ROR-beta, which is target mRNA, was analyzed by a quantitative real-time reverse transcription polymerase chain reaction (qRT-PCR) using TaqMan^{®} Gene Expression Assays (Applied Biosystems, ROR-beta: Hs00199445_m1, RN18S1 : Hs03928985_g1), THUNDERBIRD^{®} Probe qPCR Mix (TOYOBO, QPS-101), and CFX Connect Real-Time PCR Detection System (Bio-Rad). In this example, ROR-beta plasmid-treated group that was not treated with cp-asiRNA was used as a negative control, and a group having no introduced chemical modification and transfected with ROR-beta asiRNA at a concentration of 10 nM using lipofectamine RNAiMAX was used as a positive control.

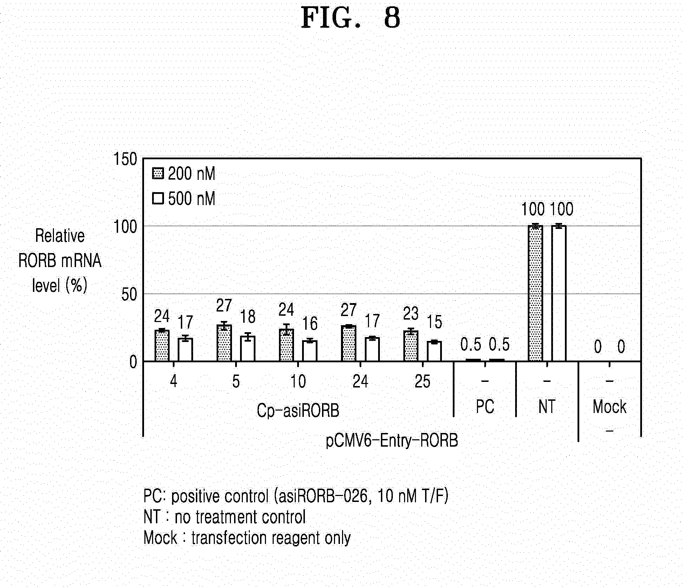

As a result, as illustrated in FIG. 8, it was confirmed that all of the 5 types of cp-asiRNA exhibited an mRNA expression inhibitory effect of 80% or more when used at a concentration of 500 nM for treatment and 70% or more when used at a concentration of 200 nM for treatment.

### 6.2. Confirmation of ROR-beta Protein Expression Inhibitory Effect

In this example, to confirm the ROR-beta protein expression inhibitory effects of the 5 types of ROR-beta cp-asiRNA (4, 5, 10, 24, and 25) derived in Example 5, an A549 cell line transiently expressing ROR-beta was treated with 200 nM or 500 nM of each cp-asiRNA, followed by western blotting to measure the expression level of the ROR-beta protein. Specifically, A549 cells were seeded into a 12-well plate at a density of 5 × 10⁴ cells/well in Ham's F-12K (Kaighn's) Medium (Gibco, 21127022) supplemented with 10% FBS, and then 200 ng of a ROR-beta plasmid was incubated in Lipofectamine 2000 (Invitrogen, 11668-019) and Opti-MEM reduced serum media (gibco, 31985- 070), and the cells were then treated therewith. After 6 hours, plate wash was performed using PBS, followed by incubation (free uptake) with 200 nM or 500 nM cp-asiRNA for 24 hours in Opti-MEM reduced serum media. The next day, the medium was replaced with Ham's F-12K (Kaighn's) Medium (10% FBS), and one day later, the expression level of the ROR-beta protein was measured in the same manner as in Example 5.2. In this example, a ROR-beta plasmid-treated group that was not treated with cp-asiRNA was used as a negative control, and a group having no introduced chemical modification and transfected with ROR-beta asiRNA at a concentration of 10 nM using lipofectamine RNAiMAX was used as a positive control.

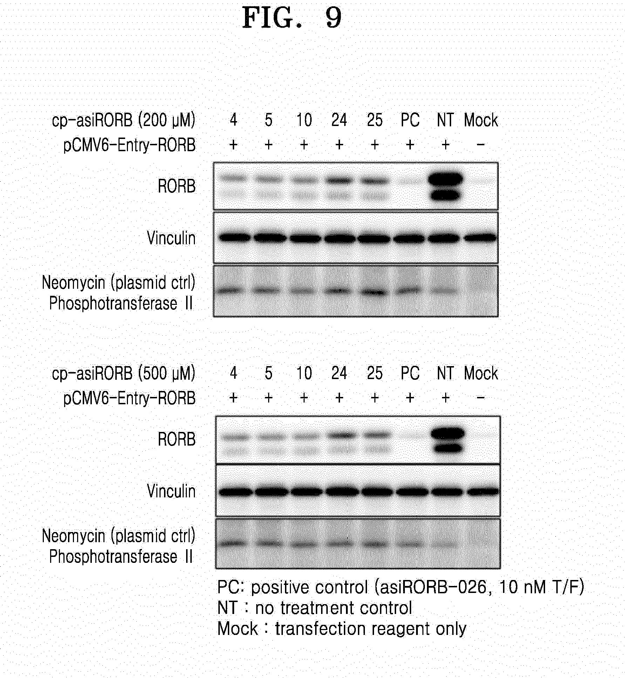

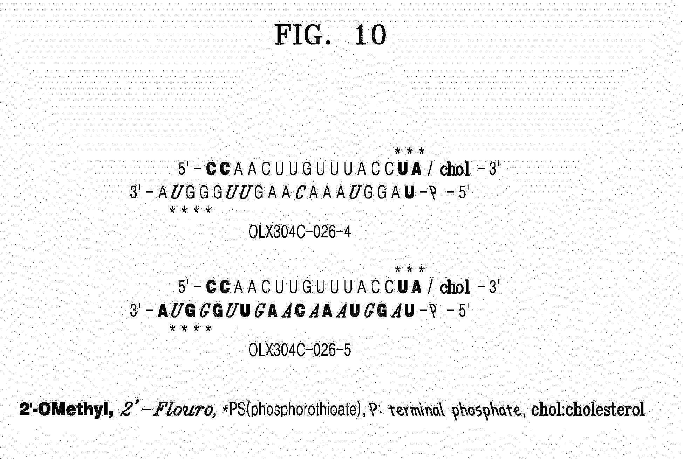

As a result, as illustrated in FIG. 9, the ROR-beta protein expression inhibitory effect according to treatment with ROR-beta cp-asiRNA was confirmed. In particular, among these, cp-asiRNAs 4 and 5 with excellent ROR-beta protein expression inhibitory effects were finally derived. FIG. 10 illustrates OLX304C-026-4 and OLX304C-026-5, which are cp-asiRNAs #4 and #5.

### Example 7: Confirmation of ROR-beta Protein Expression Inhibitory Effect in Normal Mouse Eyes

In this example, to confirm whether the two types of ROR-beta cp-asiRNA (OLX304C-026-4 and OLX304C-026-5) derived in Example 6 have high ROR-beta protein expression inhibitory effects even *in vivo,* OLX304C-026-4 or OLX304C-026-5 was administered to normal mouse eyes, followed by western blotting to measure the expression level of the ROR-beta protein. Specifically, 1 µl of OLX304C-026-4 or OLX304C-026-5 was administered at a dose of 0.6 µg/µl via intravitreal injection (IVT) in both eyes of 8-week-old male C57BI6 mice. Specifically, the administration process was as follows: after instillation of Mydrin-P eye drops (Santen, E00170121), an anesthetic was intraperitoneally injected using a 0.3 ml 31G insulin syringe (BD, 328822), followed by instillation of Alcaine eye drops (Alcon, E07370271) to carry out ocular anesthesia. Small holes were made in the sclera of the eyes with a 0.3 ml 31G insulin syringe while checking the eyes of each mouse having undergone mydriasis and anesthesia, under a surgical microscope (Leica, M844 F40). A dose of 1 µl of OLX304C-026-4 or OLX304C-026-5 was administered using a 10 µl Exmire microsyringe (Ito co, MS*NE10U) to which Microthin Dual Gauge Needle with taper 90° (35G) (Ito co., DWG.No.5-0608) was attached. Maxitrol eye ointment (Alcon, E07370151) was applied to prevent inflammation, and the mice were left on a heating mat to maintain body temperature, while waiting for them to awaken from the anesthesia. On day 7 after administration, the eyes were removed, and the retina and retinal pigmented epithelium (RPE)/choroid were separated to extract proteins. 20 µg/well of the extracted proteins was subjected to electrophoresis on an 8% SDS-polyacrylamide gel, followed by identification with ROR-beta (17635-1-AP, 1:500) and Vinculin (sc-73614, 1:2000).

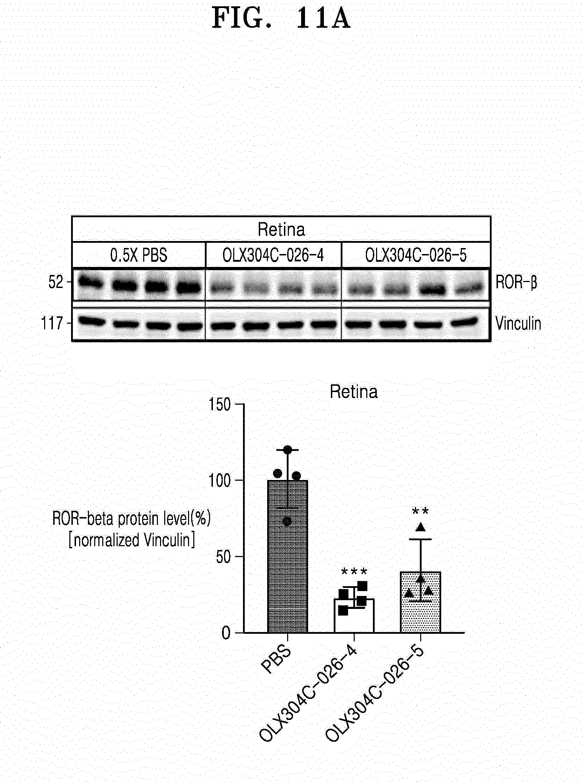

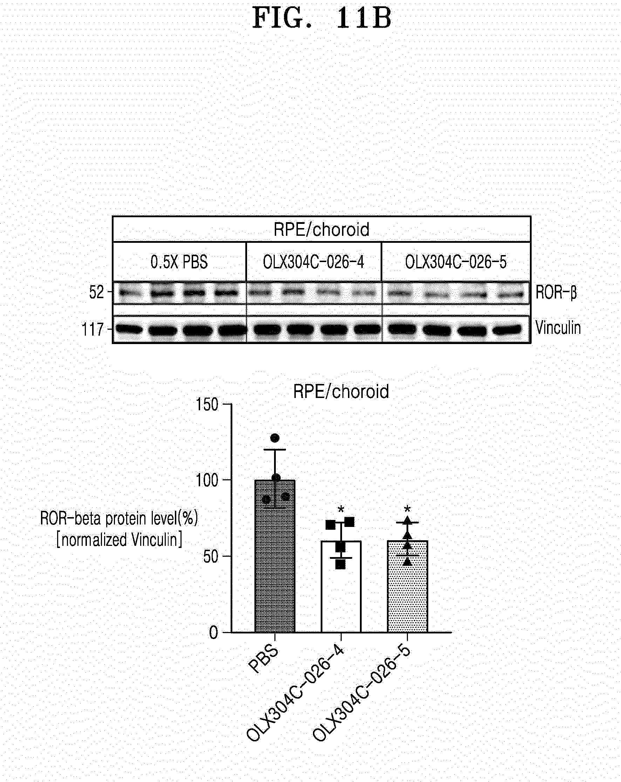

As a result, as illustrated in FIG. 11, when treated with a dose of 0.6 µg of both OLX304C-026-4 and OLX304C-026-5, a ROR-beta protein expression inhibitory effect of 60-78% was exhibited in the retinal tissue of normal mice, and similarly, a protein inhibitory effect of about 40% was also shown in the RPE/choroid layer.

### Example 8: Confirmation of ROR-beta mRNA Expression Inhibitory Effect according to Dose in Normal Mouse Eyes

In this example, to confirm high *in vivo* ROR-beta mRNA expression inhibitory effects of OLX304C-026-4, which is one of the two types of ROR-beta cp-asiRNA derived in Example 6, and OLX304C-026-26, which was further synthesized by adding palmitic acid instead of cholesterol to the 3' end of a sense strand of OLX304C-026-4, OLX304C-026-4 or OLX304C-026-26 was administered to normal mouse eyes, followed by real-time PCR to measure the expression level of ROR-beta mRNA. Specifically, the administration process was performed in the same manner as in Example 7, except that 1 µl of OLX304C-026-4 was administered at doses of 0.1, 0.4, and 1.0 µg/µl and 1 µl of OLX304C-026-26 was administered at doses of 0.4, 1.0, and 4.0 µg/µl to both eyes of 7-week-old male C57BI6 mice via intravitreal injection (IVT), and on day 7 after administration, the eyes were removed and RNA was extracted from retinal tissues. RNA extraction was performed using the RNeasy Plue Mini Kit (QiAGEN, 74136) according to the manufacturer's protocol. The extracted RNA was used to synthesize cNDA using the High-Capacity cDNA Reverse Transcription Kit (Applied Biosystems^{™}, 4368814) according to the manufacturer's protocol, and then the expression level of ROR-beta, which is target mRNA, was analyzed by a quantitative real-time reverse transcription polymerase chain reaction (qRT-PCR) using a TB Green^{®} Premix Ex Taq^{™} II (TaKaRa, RR820B) product and CFX Connect Real-Time PCR Detection System (Bio-Rad). qRT-PCR was carried out using ROR-beta primers (F: 5'- TGCCCAAGTCCGAAGGTTATT-3' (SEQ ID NO: 125), R: 5'- CCATGCCAGCTGATGGAGTT-3' (SEQ ID NO: 126), and GAPDH primers (F: 5'-GGGTGTGAACCACGAGAAAT-3' (SEQ ID NO: 127), R: 5'-GTCATGAGCCCTTCCACAAT-3' (SEQ ID NO: 128)).

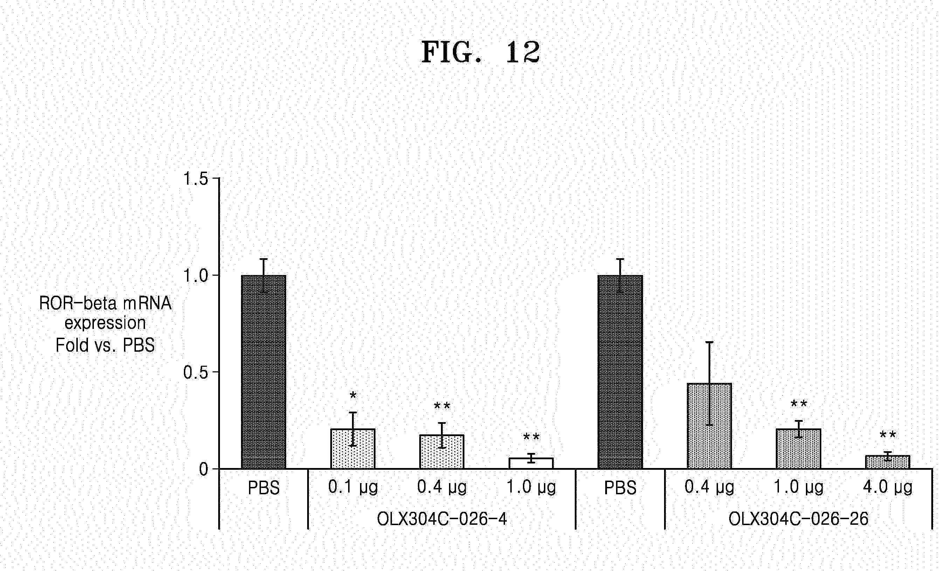

As a result, as illustrated in FIG. 12, it was confirmed that both of the two types of cp-asiRNA showed ROR-beta mRNA expression inhibitory effects in a dose-dependent manner, and OLX304C-026-4 showed an expression inhibitory effect of about 97% at 1.0 µg, and OLX304C-026-26 showed an expression inhibitory effect of about 87% at 4.0 µg.

**[Table 5]**

| **No.** | **Name** | **Sequence (5' → 3')** | |
|---|---|---|---|
| 68 | 0LX304C-026-26 | sense | mCmCAACUUGUUUACC∗mU∗mA∗PA |
| | | antisense | P-mUAGGfUAAAfCAAGfUfUG∗G∗G∗fU∗A |

In Table 5, chemical modifications indicated by "*", "m", "f", and "PA" are the same as shown in Table 6 below.

**[Table 6]**

| **Notation** | **Chemical modification** |
|---|---|
| * | **phosphorothioate bond** |
| m | **2'-O-methyl** |
| f | **2'-fluoro** |
| PA | **palmitic acid** |

### Example 9: Confirmation of Duration of ROR-beta mRNA Expression Inhibitory Effect in Normal Mouse Eyes

In this example, to confirm the duration of the ROR-beta mRNA expression inhibitory effect of OLX304C-026-4, which is one of the two types of ROR-beta cp-asiRNA derived in Example 7, OLX304C-026-4 was administered to normal mouse eyes, followed by real-time PCR to measure the expression level of ROR-beta mRNA. Specifically, the experiment was carried out in the same manner as in Example 8, except that 1 µl of OLX304C-026-4 was administered at a dose of 1.0 µg/µl or 2.0 µg/µl to the eyes of 7-week-old male C57BI6 mice via intravitreal injection (IVT), and on day 14, day 21 or day 28 after administration, the eyes were removed and RNA was extracted from retinal tissues.

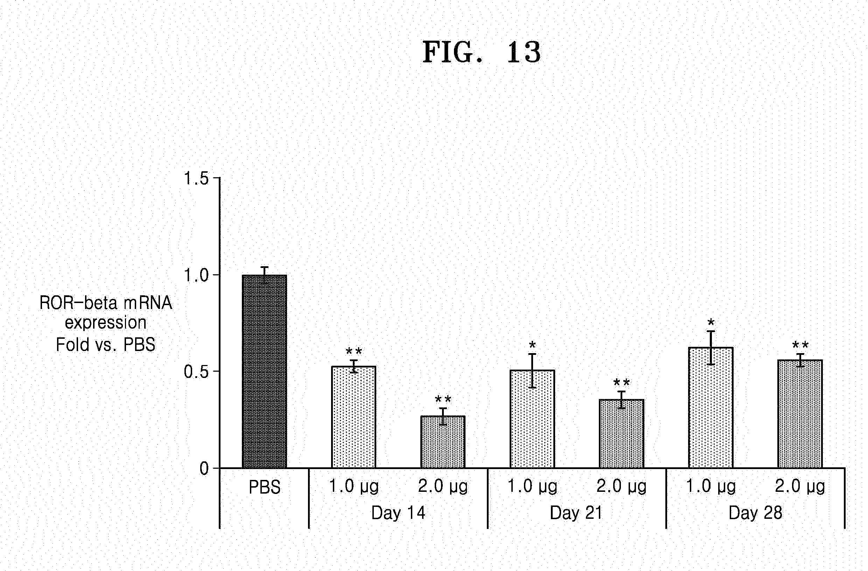

As a result, as illustrated in FIG. 13, after administration of 2 µg, OLX304C-026-4 maintained a ROR-beta mRNA expression inhibitory effect of about 44% up to day 28. The tendency to be maintained for a long period of time was more effective with administration at 2 µg than at 1 µg, and it was confirmed that similar ROR-beta mRNA expression inhibitory effects were shown in the two doses on day 28 after administration. It was also confirmed that a ROR-beta mRNA expression inhibitory effect of about 50% or more was exhibited up to three weeks when administered at 1 µg.

### Example 10: Confirmation of Therapeutic Effect in Retinitis Pigmentosa (RP) Disease Mouse Model

In this example, an efficacy test was conducted in a retinitis pigmentosa disease mouse model via administration at the optimal ROR-beta mRNA expression inhibitory dose verified in Example 8. Specifically, a heterozygote of Rho P23H knock-in mouse known as a representative retinitis pigmentosa disease model mouse was used in the experiment. According to the results of Example 8, 1.0 µg of OLX304C-026-4 and 4.0 µg of OLX304C-026-26 were administered, and then on day 21 after administration, ocular tissue analysis and an electroretinogram (ERG) test were performed to confirm therapeutic effects. Intraocular injection of OLX304C-026-4 and OLX304C-026-26 was performed in the same manner as in Example 8, and H&E staining was performed using an automatic slide stainer using tissue sections obtained through paraffin sectioning. For ERG imaging, dark adaptation was performed in a dark room one day before mice were photographed, and imaging was performed while maintaining a dark state. Mydriasis and anesthesia were performed in the same manner as in Example 8, and a ground electrode was fixed to the skin just above the tail and a reference electrode was fixed to the middle of the forehead. 2% Hycell^{™} sterilization solution (Samil Pharmaceutical, A05050401) was applied to the eyeballs, and the eyeballs were positioned to be into good contact with a Ganzfeld ERG (Phoenix, MICRON Ganzfeld ERG) device, followed by photographing sequentially from low-intensity light stimulation to high-intensity light stimulation.

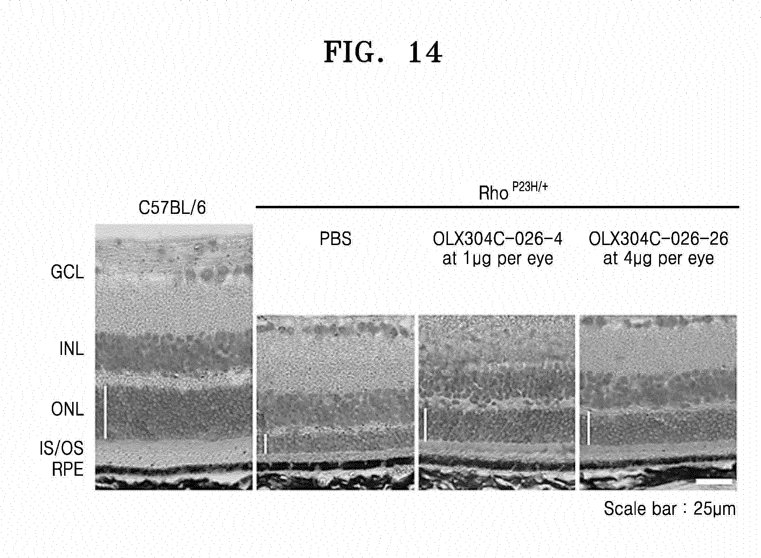

As illustrated in FIG. 14, as a result of comparing the thicknesses of respective layers through H&E staining of eye tissue in OLX304C-026-4 and OLX304C-026-26, it was confirmed that the thickness of the eye tissue was significantly increased in the case of OLX304C-026-4 or OLX304C-026-26 administration. In particular, an increase in the thickness of an outer nuclear layer (ONL) where photoreceptor cells are mainly located seems to be related to the restoration of visual function, compared to the control, through which it can be confirmed that, when OLX304C-026-4 or OLX304C-026-26 was administered, the ONL layer is protected from collapsing.

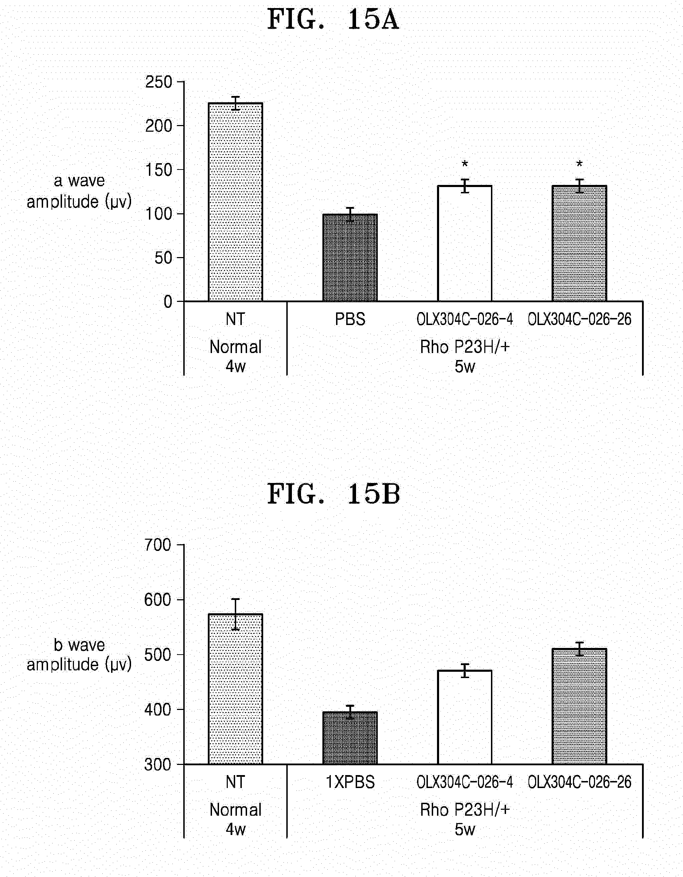

Similarly, referring to FIG. 15 illustrating the ERG measurement results, it was also confirmed that, in the group administered with OLX304C-026-4 or OLX304C-026-26, damaged a-waves were significantly recovered by about 25% and 25%, respectively, and damaged b-waves were significantly recovered by about 42% and 65%, respectively, compared to the control. This is a strategy for transforming rod cells with various genetic defects into cone cells in patients with retinitis pigmentosa, which demonstrates that visual impairment can be delayed or treated through intravitreal administration of OLX304C-026-4 or OLX304C-026-26 targeting ROR-beta.

While specific embodiments of the present disclosure have been described in detail, it will be apparent to those of ordinary skill in the art that these detailed descriptions are exemplary embodiments only and are not intended to limit the scope of the present disclosure. Therefore, the substantial scope of the present disclosure will be defined by the appended claims and equivalents thereto.

## Claims

1. An RNAi-inducing nucleic acid molecule comprising: an antisense strand comprising a sequence complementary to mRNA encoding retinoid-related orphan nuclear receptor (ROR)-beta; and a sense strand forming complementary bonds with the antisense strand, wherein the 5' end of the antisense strand and the 3' end of the sense strand form a blunt end.

2. The RNAi-inducing nucleic acid molecule of claim 1, wherein the sense strand has a length of 15 nt to 17 nt, and the antisense strand has a length of 18 nt to 23 nt.

3. The RNAi-inducing nucleic acid molecule of claim 1, wherein the sense strand is selected from the group consisting of SEQ ID NOs: 27, 29, 51, 83, 85, 91, 95, 97, 103, 105, 107, 109, and 115.

4. The RNAi-inducing nucleic acid molecule of claim 3, wherein the sense strand is selected from the group consisting of SEQ ID NOs: 27, 29, 51, and 109.

5. The RNAi-inducing nucleic acid molecule of claim 1, wherein the antisense strand is selected from the group consisting of SEQ ID NOs: 28, 30, 52, 84, 86, 92, 96, 98, 104, 106, 108, 110, and 116.

6. The RNAi-inducing nucleic acid molecule of claim 5, wherein the antisense strand is selected from the group consisting of SEQ ID NOs: 28, 30, 52, and 110.

7. The RNAi-inducing nucleic acid molecule of claim 1, wherein the sense strand or the antisense strand of the RNAi-inducing nucleic acid molecule comprises at least one chemical modification selected from:
substitution of an -OH group at a 2' carbon position of a sugar structure of a nucleotide with methyl (-CH₃), methoxy (-OCH₃), -NH₂, fluorine (-F), -O-2-methoxyethyl-O-propyl, -O-2-methylthioethyl, -O-3-aminopropyl, or -O-3-dimethylaminopropyl;
substitution of oxygen of a sugar structure of a nucleotide with sulfur;
modification of a nucleotide linkage, with phosphorothioate, boranophosphate, or methyl phosphonate;
modification into a peptide nucleic acid (PNA) form, a locked nucleic acid (LNA) form, or an unlocked nucleic acid (UNA) form; and
linkage of a phosphate group, a lipophilic compound, or a cell-penetrating peptide.

8. The RNAi-inducing nucleic acid molecule of claim 7, wherein the lipophilic compound is selected from the group consisting of cholesterol, tocopherol, stearic acid, retinoic acid, decosahexaenoic acid (DHA), palmitic acid, linoleic acid, linolenic acid, and long-chain fatty acids having 10 or more carbon atoms.

9. The RNAi-inducing nucleic acid molecule of claim 7, wherein the sense strand comprises at least one chemical modification selected from:
modification of 2 to 4 nucleotide linkages adjacent to the 3' end with phosphorothioate, boranophosphate, or methyl phosphonate;
substitution of an -OH group at a 2' carbon position of a sugar structure of at least two nucleotides with methyl (-CH₃), methoxy (-OCH₃), -NH₂, fluorine (-F), -0-2-methoxyethyl-O-propyl, -O-2-methylthioethyl, -O-3-aminopropyl, or -0-3-dimethylaminopropyl; and
linkage of a lipophilic compound or a cell-penetrating peptide to the 3' end.

10. The RNAi-inducing nucleic acid molecule of claim 7, wherein the antisense strand comprises at least one chemical modification selected from:
modification of 3 to 5 nucleotide linkages adjacent to the 3' end, with phosphorothioate, boranophosphate, or methyl phosphonate;
substitution of an -OH group at a 2' carbon position of a sugar structure of at least two nucleotides with methyl (-CH₃), methoxy (-OCH₃), -NH₂, fluorine (-F), -0-2-methoxyethyl-O-propyl, -O-2-methylthioethyl, -O-3-aminopropyl, or -0-3-dimethylaminopropyl; and
linkage of a phosphate group or a cell-penetrating peptide to the 5' end.

11. The RNAi-inducing nucleic acid molecule of claim 7, wherein the RNAi-inducing nucleic acid molecule comprises at least one chemical modification selected from:
a modification in which an -OH group at a 2' carbon position of a sugar structure of at least two nucleotides of the sense strand or the antisense strand is substituted with methoxy (-OCH₃) or fluorine (-F);
modification of 10% or more of nucleotide linkages of the sense or antisense strand with phosphorothioate;
Linkage of cholesterol or palmitic acid to the 3' end of the sense strand; and
Linkage of a phosphate group to the 5' end of the antisense strand.

12. The RNAi-inducing nucleic acid molecule of claim 7, wherein the RNAi-inducing nucleic acid molecule comprises any one antisense strand selected from:
(a) P-mUAGGfUAAAfCAAGfUfUG*G*G*fU*A; and
(b) P-mUfAmGfGmUfAmAfAmCfAmAfGmUfUmG*fG*mG*fU*mA,
wherein * denotes a modification into a phosphorothioate linkage, m denotes a substitution with 2'-OCH₃, f denotes a substitution of 2'-F, and P denotes linkage of a 5'-phosphate group.

13. The RNAi-inducing nucleic acid molecule of claim 7, wherein the RNAi-inducing nucleic acid molecule comprises any one sense strand selected from:
(A) mCmCAACUUGUUUACC*mU*mA*chol;
(B) mCCmAAmCUmUGmUUmUAmCC*mU*A*chol;
(C) mCfCmAfAmCfUmUfGmUfUmUfAmCfC*mU*fA*chol; and
(D) mCmCAACUUGUUUACC*mU*mA*PA,
wherein * denotes a modification into a phosphorothioate linkage, m denotes a substitution with 2'-OCH₃, chol denotes linkage of 3'-cholesterol, and PA denotes linkage of 3'-palmitic acid.

14. The RNAi-inducing nucleic acid molecule of claim 7, wherein the RNAi-inducing nucleic acid molecule has a cell-penetrating ability.

15. A pharmaceutical composition for ameliorating or treating a retinal disease, comprising the RNAi-inducing nucleic acid molecule according to any one of claims 1 to 14 as an active ingredient.

16. The pharmaceutical composition of claim 15, wherein the retinal disease is any one selected from the group consisting of Usher syndrome, Stargardt disease, Bardet-Biedl syndrome, Best's disease, choroideremia, gyrate-atrophy, retinitis pigmentosa, retinal macular degeneration, Leber Congenital Amaurosis (Leber's Hereditary Optic Neuropathy), blue-cone monochromacy (BCM), retinoschisis, Malattia Leventinese (ML), Oguchi disease, and Refsum disease.
